(19) Europäisches Patentamt
European Patent Office
Office européen des brevets

(11) EP 0 749 473 B1

(12) EUROPEAN PATENT SPECIFICATION

(45) Date of publication and mention
of the grant of the patent:
**12.10.2005 Bulletin 2005/41**

(51) Int Cl.$^7$: **C12N 9/42**

(21) Application number: **95911227.7**

(86) International application number:
**PCT/DK1995/000108**

(22) Date of filing: **08.03.1995**

(87) International publication number:
**WO 1995/024471 (14.09.1995 Gazette 1995/39)**

(54) **NOVEL ALKALINE CELLULASES**

NEUARTIGE ALKALISCHE ZELLULASEN

NOUVELLES CELLULASES ALCALINES

(84) Designated Contracting States:
**DE DK ES FR GB IT NL**

(30) Priority: **08.03.1994 DK 27094**
**30.03.1994 DK 36594**

(43) Date of publication of application:
**27.12.1996 Bulletin 1996/52**

(73) Proprietor: **Novozymes A/S**
**2880 Bagsvaerd (DK)**

(72) Inventors:
• **SCHÜLEIN, Martin**
**DK-2880 Bagsvaerd (DK)**
• **ROSHOLM, Peter**
**DK-2880 Bagsvaerd (DK)**
• **NIELSEN, Jack Bech**
**DK-2880 Bagsvaerd (DK)**
• **HANSEN, Svend Aage**
**DK-2880 Bagsvaerd (DK)**
• **VON DER OSTEN, Claus**
**DK-2880 Bagsvaerd (DK)**

(56) References cited:
EP-A- 0 137 280          EP-A- 0 495 257
EP-A- 0 495 258          EP-A- 0 540 784
WO-A-85/04672          WO-A-91/17243
WO-A-91/17244          WO-A-93/05226
WO-A-94/07998          US-A- 5 246 853
US-A- 5 298 405

• TIBTECH, Volume 7, Sept 1989, EDGAR ONG et al., "The Cellulose-binding Domains of Cellulases: Tools for Biotechnology", page 239.

• BIOTECHNOLOGY, Volume 5, January 1987, JANELLE N. VAN ARSDELL et al., "Cloning, Characterization, and Expression in Saccharomyces Cerevisiae of Endoglucanase i from Trichoderma Reesei", page 60.
• DIALOG INFORMATION SERVICES, File 154, Medline Dialog accession no. 06132822, Medline accession no. 87106822, PENTTILA M. et al., "Homology Between Cellulase Genes of Trichoderma Reesei: Complete Nucleotide Sequence of the Endoglucanase I Gene"; & GENE (NETHERLANDS) 1986, 45 (3) p 253-63.
• DIALOG INFORMATION SERVICES, File 351, World Patent Index 81-95, Dialog accession no. 009814968, WPI accession no. 94-094824/12, NIPPON KAMI PULP KENKYUSHO KK, "Cellulase Used for Enzymatic Saccharification of Cellulosic Materials, etc. - Prepd. from Microbe of Trichoderma Genus and has Cellulolytic Activity"; & JP,A,06 038 747, 15-02-94, 9412 (Basic).
• DIALOG INFORMATION SERVICES, File 357, Derwent Biotechnology Abs, Dialog accession no. 071219, DBA accession no. 88-01567, RIKAGAKU-RES. INST., "Cellulase Gene - Obtained from Bacillus sp. and Expressed in Escherichia Coli"; & JP,A,62 232 386 (Kokai), 12-10-87.
• DIALOG INFORMATION SERVICES, File 357, Derwent Biotechnology Abs, Dialog accession no. 086768, DBA accession no. 89-04759, RAO M. et al., "Glucohydrolase from Penicillium Funiculosum - Exo-1,4-beta-Glucosidase Purification and Characterization; Cellulase Complex"; & APPL. MICROBIOL. BIOTECHNOL. (30, 2, 130-34) 1989.

- **DIALOG INFORMATION SERVICES, File 55, Biosis, Dialog accession no. 10066786, Biosis accession no. 95066786, GOTO M. et al., "An Avicel-Affinity Site in an Avicel-Digesting Exocellulase from a Trichoderma-Viride Mutant"; & BIOSCI BIOTECHNOL BIOCHEM 56 (10), 1992, 1523-1528.**
- **DIALOG INFORMATION SERVICES, File 357, Derwent Biotechnology Abs, Dialog accession no. 125563, DBA accession no. 91-13205, BIELY P. et al., "The Endo-1,4-beta-Glucanase-I from Trichoderma Reesei: Action on beta-1,4-Oligomers and Polymers Derived from D-glucose and D-xylose-cellulase"; & EUR. J. BIOCHEM. (200, 1, 157-63) 1991.**
- **NATIONAL LIBRARY OF MEDICINE, File Medline, NLM accession no. 94028932, LAO G., "DNA Sequences and Expression in Streptomyces Lividans of an Exoglucanase Gene and an Endoglucanase Gene from Thermomonospora Fusca"; & APPL ENVIRON MICROBIOL 1993 Sep; 59 (9): 3032-43.**
- **NATIONAL LIBRARY OF MEDICINE, File Medline, NLM accession no. 90197611, BHAT K.M., "Study of the Mode of Action and Site-Specificity of the Endo-(1---4)-beta-D-Glucanases of the Fungus Penicillium Pinophilum with Normal, 1-3H-Labelled, Reduced and Chromogenic Cello-Oligosaccharides"; & BIOCHEM J 1990 Mar 1; 266(2):371-8.**
- **DIALOG INFORMATION SERVICES, File 55, Biosis, Dialog accession no. 4953447, Biosis accession no. 80080758, OKADA G., "Purification and Properties of a Cellulase from Aspergillos-Niger"; & AGRIC BIOL CHEM 49 (5), 1985, 1257-1266.**

Remarks:

The file contains technical information submitted after the application was filed and not included in this specification

**Description**

The present invention relates to a cellulase capable of removing soil from fabric, a detergent composition comprising the cellulase, a method of treating soiled fabric with the cellulolytic enzyme, and use of the cellulase e.g. in detergent compositions, in fabric softeners, for colour clarification of textile fabrics (removal of fluffs and pills), for preventing backstaining in washing of fabric, for soil removal, for deinking of used paper, and for pulp recycling.

BACKGROUND OF THE INVENTION

Repeated washing of fabrics, especially cellulose containing fabrics, generally causes a harshness in the fabric used. The use of cellulases, i.e. cellulolytic enzymes, for harshness reduction of cellulose containing fabrics, e. g. cotton, was suggested and demonstrated a long time ago.
The practical exploitation of cellulases has, to some extent, been set back by the nature of the known cellulase preparations which are often complex mixtures of a variety of single cellulase components, and which may have a rather low specific activity. It is difficult to optimise the production of single components in multiple enzyme systems and thus to implement industrial cost-effective production of cellulases, and their actual use has been hampered by difficulties arising from the need to employ rather large quantities of the enzymes to achieve the desired effect.
The drawbacks of previously suggested cellulases may be remedied by using single-component enzymes selected for a high specific activity. Single-component cellulases are described in, e.g. WO 91/17243, WO 91/17244 and WO 91/10732.
E.g. in WO 91/17244 is disclosed a cellulose-degrading enzyme (a cellulase) derivable from a fungus other than *Trichoderma* or *Phanerochaete* which comprises a carbohydrate binding domain homologous to a terminal A region of *Trichoderma reesei* cellulases, the carbohydrate binding domain being capable of effecting binding of the enzyme to an insoluble cellulosic substrate, which may be employed for textile treatment, e.g. for reducing the harshness of cotton-containing fabrics and for soil removal and colour clarification of fabrics. In example 3 and fig. 13 is disclosed the preparation of a *Fusarium oxysporum* C-family endoglucanase and the DNA sequence and derived amino acid sequence thereof, respectively. Later it was found that the disclosed amino acid sequence was not correct; the corrected sequence is published in Sheppard, P.O., Grant, F.J., Oort, P.J., Sprecher, C.A., Foster, D.C., Hagen, F.S., Upshall, A., Mcknight, G.L. and Ohara, P.J.: The use of conserved cellulase family-specific sequences to clone cellulase homolog cdnas from fusarium-oxysporum. Gene. 150:163-167, 1994. In example 4 and Fig. 14A-E is disclosed the preparation of a *Humicola insolens* endoglucanase 1 (EG I) and the DNA sequence and derived amino acid sequence thereof, respectively. Further, in example 4 (page 32, line 1 to 5) is described the construction of expression plasmid of a truncated EG I (denoted EG I') wherein the last 13 amino acids of the coding region were eliminated and the altering of Val to Leu in position 421 (position 401 in the sequence of the enzyme). The gist of the invention disclosed in WO 91/17244 is to provide a cellulase which, besides the enzyme core, has a carbohydrate binding domain (CBD) which is homologous to the A region of *Trichoderma reesei* cellulases, since the function of the CBD in the enzyme molecule was believed to be to mediate binding to solid substrates including cellulose and consequently to enhance the activity of such enzymes towards such substrates.
The problem underlying the present invention is to obtain single-component endoglucanases having enhanced enzyme activity in the alkaline pH range, while at the same time exerting a moderate cellulolytic action on the cellulosic substrate. In other words, the endoglucanase should neither destroy the cellulosic substrate as such. For example, when the substrate is a cellulosic fabric, the used endoglucanase should not result in a substantial tensile strength loss of the fabric. The enhanced alkaline activity of the enzyme is also essential, since most applications of endoglucanases advantageously take place in the alkaline pH range.

SUMMARY OF THE INVENTION

Surprisingly, it has now been found that certain cellulases which do not comprise a carbohydrate binding domain, i.e. essentially consist of the core enzyme, or which at least do not comprise a carbohydrate binding domain which is homologous to the A region of *Trichoderma reesei* cellulases, may have an enhanced activity. This may for example result in improved soil removal from fabrics.
More specifically, it has been found that cellulases selected from the group consisting of cellulases classified in family 7 as described in Henrissat, B. et al.: Biochem. J. (1993), **293,** p. 781-788, and cellulase variants derived from a parent cellulase classified in family C, comprising a core and optionally a C-terminal link consisting of 10 amino acids at the most may perform excellent in detergents with respect to soil removal in comparison with the known cellulases.
In one aspect, the invention relates to endoglucanases which may be truncated, e.g. genetically truncated, variants of known endoglucanases, and the use thereof e.g. for washing, cleaning, deinking and pulping purposes.
In another aspect, the invention relates to cellulases having high activity on cellotriose in the presence of a detergent matrix, high dispersing action on carbon black, and high alkaline activity on acid swollen cellulose at pH 8.

The present endoglucanases are useful e.g. for soil removal and may thus be applied to detergent compositions, detergent additives and/or fabric softeners.

Other uses of the present endoglucanases are for colour clarification of textile fabrics (removal of fluffs and pills), for preventing backstaining in washing of fabric, for soil removal, for deinking of used paper, and for pulp recycling.

DETAILED DESCRIPTION OF THE INVENTION

In the present specification and claims, the term "cellulase" denotes an enzyme that hydrolyses cellulose. The cellulase may be a component occurring in a cellulase system produced by a given microorganism, such a cellulase system mostly comprising several different cellulase enzyme components including those usually identified as e.g. cellobiohydrolases, exo-cellobiohydrolases, endoglucanases, $\beta$-glucosidases. Alternatively, the cellulase may be a single component, i.e. a component essentially free of other cellulase components usually occurring in a cellulase system produced by a given microorganism, the single component being a recombinant component, i.e. produced by cloning of a DNA sequence encoding the single component and subsequent cell transformed with the DNA sequence and expressed in a host.

The host is preferably a heterologous host, but the host may under certain conditions also be the homologous host.

In a preferred embodiment of the invention, the cellulase is an endoglucanase.

The term "soil removal" or "particulate soil removal", as used herein, refers to enhanced cleaning of cellulose-containing fabrics or garment, e.g. cotton, contaminated by particles of soil or of other insoluble matter entrapped by microfibrills spreading out on the fibre surface.

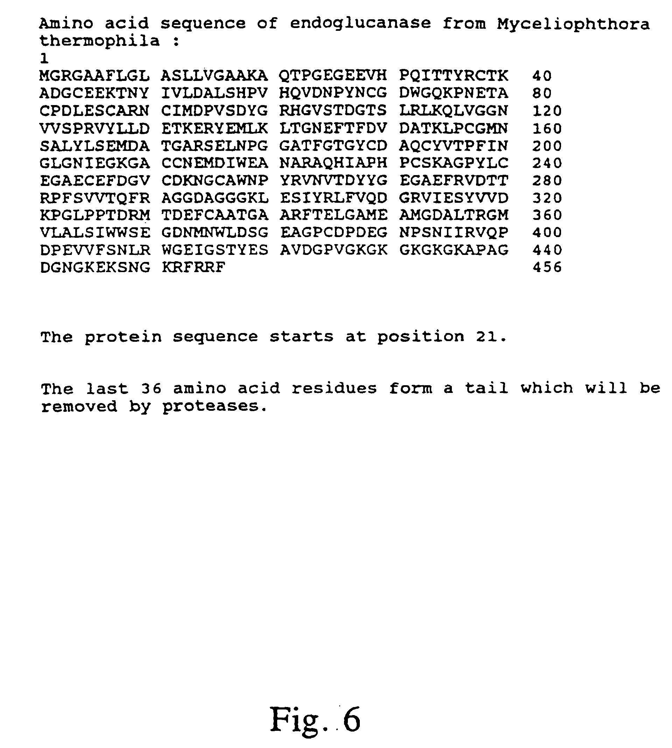

In the present context, the term "homologous" or "homologous sequence" is intended to indicate an amino acid sequence differing from that of Figure 6 by one or more amino acid residues. The homologous sequence may be one resulting from modification of an amino acid sequence shown in these figures, e.g. involving substitution of one or more amino acid residues at one or more different sites in the amino acid sequence, deletion of one or more amino acid residues at either or both ends of the enzyme or at one or more sites in the amino acid sequence, or insertion of one or more amino acid residues at one or more sites in the amino acid sequence. The modification of the amino acid sequence may suitably be performed by modifying the DNA sequence encoding the enzyme, e.g. by site-directed or by random mutagenesis or a combination of these techniques in accordance with well-known procedures. Alternatively, the homologous sequence may be one of an enzyme derived from another origin than the cellulases corresponding to the amino acid sequence shown in Figure 6. Thus, "homologue" may e.g. indicate a polypeptide encoded by DNA which hybridizes to the same probe as the DNA coding for the cellulase with the amino acid sequence in question under certain specified conditions (such as presoaking in 5xSSC and prehybridising for 1 h at ~40°C in a solution of 20% formamide, 5xDenhard't's solution, 50 mM sodium phosphate, pH 6.8, and 50 $\mu$g of denatured sonicated calf thymus DNA, followed by hybridization in the same solution supplemented with 100 $\mu$M ATP for 18 h at ~40°C). The homologous sequence will normally exhibit a degree of homology (in terms of identity) of at least 50%, such as at least 60%, 65%, 70%, 75%, 80%, 85%, 90% or even 95% with the amino acid sequence shown in Figure 6.

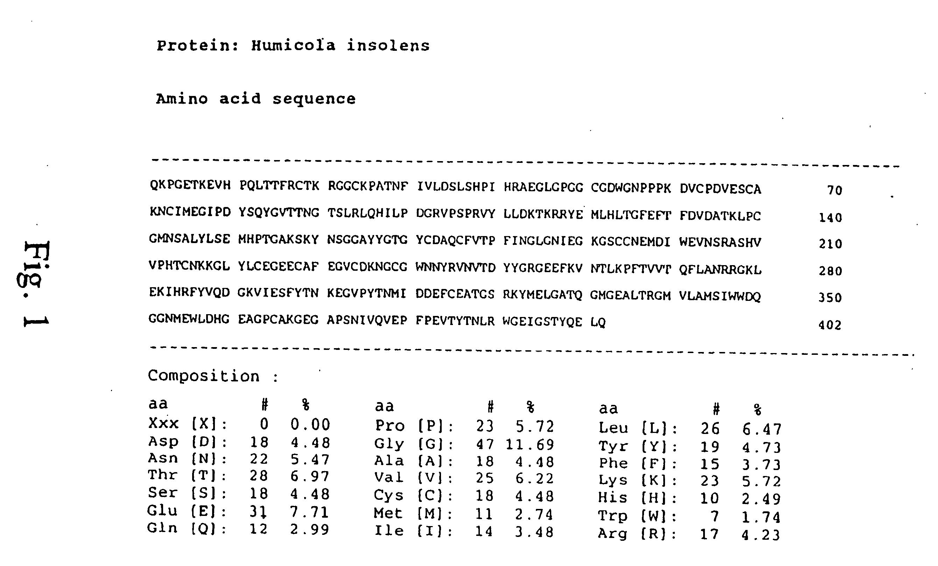

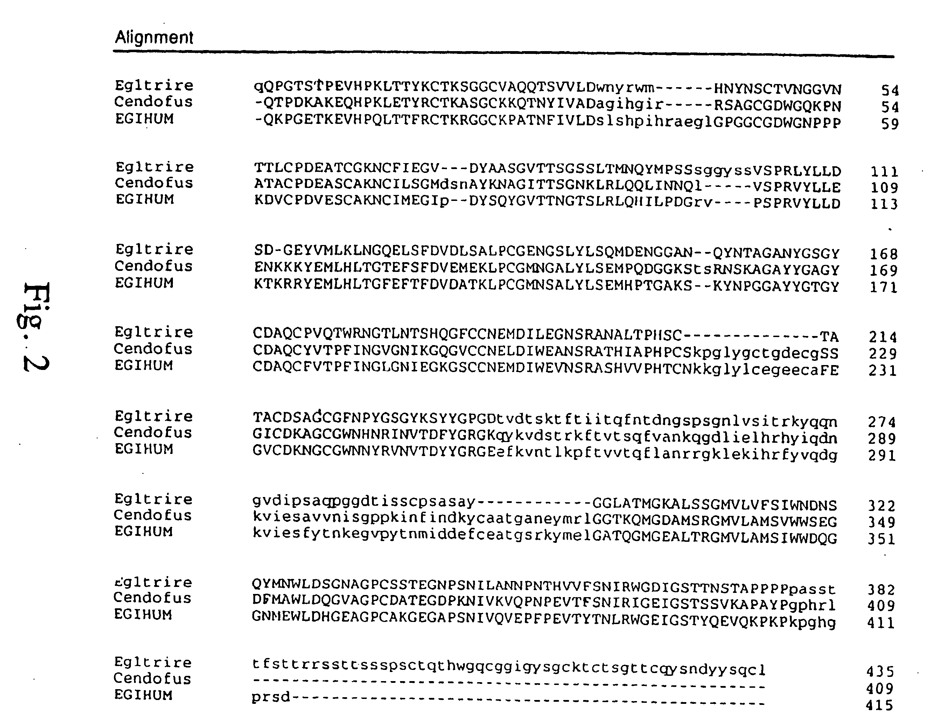

The present invention relates to a cellulase which is selected from the group consisting of cellulases classified in family 7 as described in Henrissat, B. et al.: Biochem. J. (1993), **293**, p. 781-788, and cellulase variants derived from a parent cellulase classified in family C, wherein the cellulase comprises a core and optionally a C-terminal link consisting of 10 amino acids at the most, provided that the cellulase is different from the endoglucanase having the amino acid sequence listed in Figure 1.

The classification by Henrissat is a new classification system for glycosyl hydrolases based on sequence comparisons and hydrophobic cluster analyses which have shown that the catalytic domains of glycosyl hydrolases fall into 45 distinct families, of which 11 (originally denoted A-K) contain enzymes with cellulolytic activity.

Thus far, structures of the catalytic domains of cellulases from four families have been published:

Cellobiohydrolase II (CBH II) from Trichoderma reesei (Rouvinen et al. 1990) and endocellulase E2 from Thermomonospora fusca (Sapezio et al. 1993) from family 6(B),
Cellobiohydrolase I (CBH I) from T. reesei (Divne et al. 1994) from family 7 (C),
CelA from Clostridium thermocellum (Juy et al. 1992) from family 9(E); and the endoglcucanase V from H. insolens (Davies et al. 1993) from family 45(K).

The cellulases of the invention may be obtainable by or derived from a strain of *Humicola, Trichoderma, Myceliophthora, Penicillium, Irpex, Aspergillus, Scytalidium* or *Fusarium*, preferably from a strain of *Humicola, Trichoderma, Myceliophthora, Scytalidium* or *Fusarium,* more preferably from a strain of *Humicola insolens, Fusarium oxysporum, Myceliophthora thermophila* or *Trichoderma reesei.*

The cellulase of the invention can advantageously have one or more insertions of between 1 and 25, preferably between 1 and 20, amino acid residues; preferably the insertion is relative to EG1-Tat position 230-240 in EG1-H and EG1-F and part of which is located within 10Å of the active site residues E197, D199 and E202.

In another aspect, the cellulase of the invention has a substrate binding cleft with a depth of at least 12Å, preferably 15Å, when measuring between C$\alpha$ atoms of the active site residues located at the bottom of the cleft and C$\alpha$ atoms of residues located at the rim of the substrate binding cleft immediately above the active site residues.

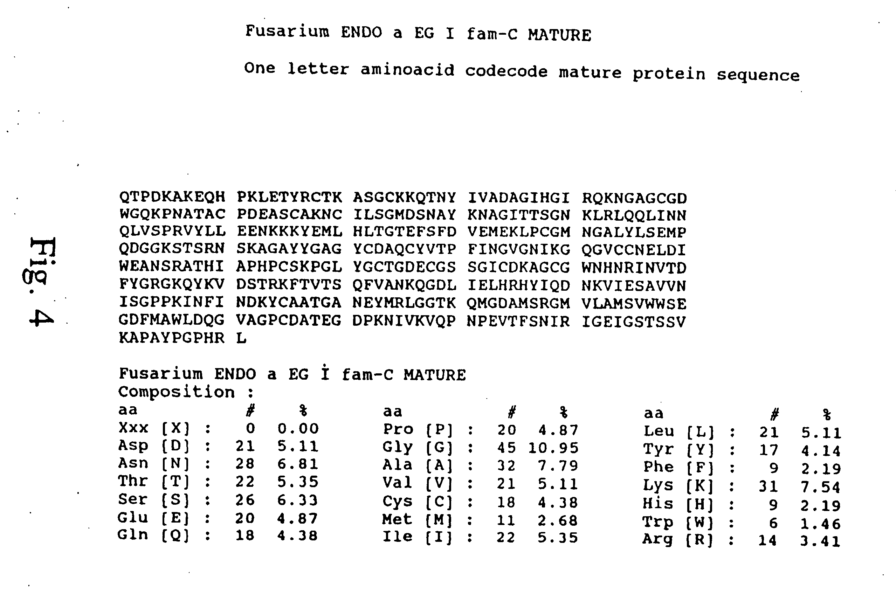

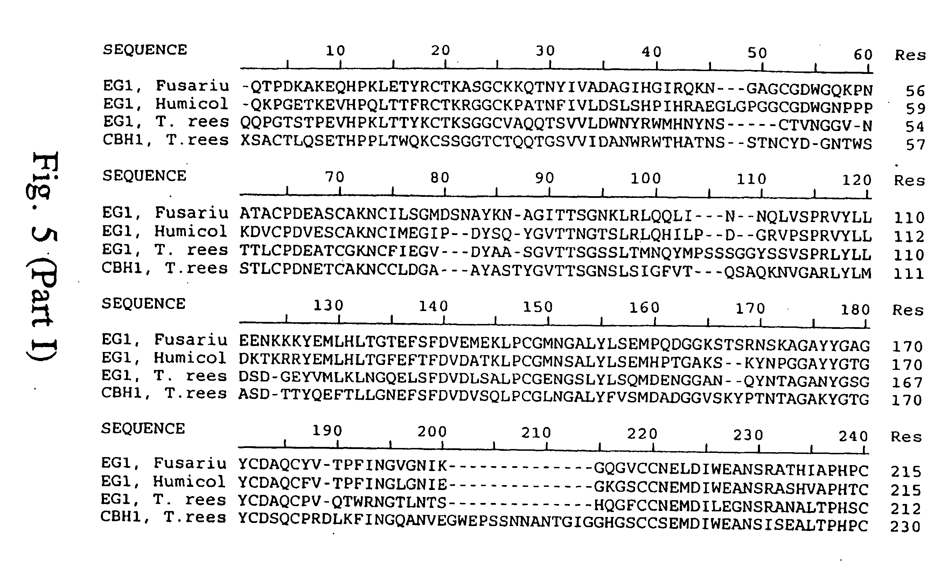

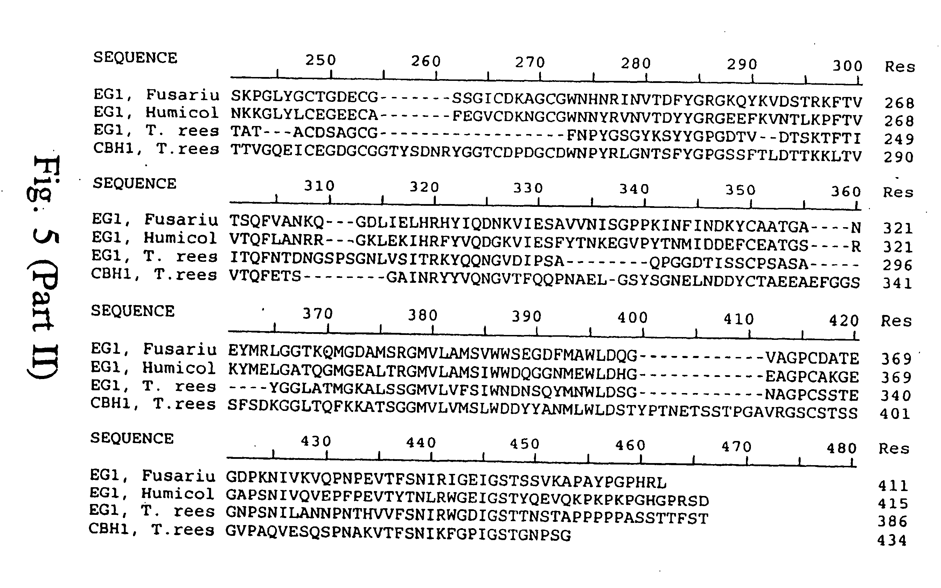

According to the invention, cellulases which essentially consists of the core and optionally a C-terminal link having 10 amino acids at the most may perform excellent for particulate soil removal when used for washing/laundry or fabric softening purposes. In Figure 1 is disclosed the amino acid sequence of a genetically truncated endoglucanase from *Humicola insolens* of 402 amino acid residues which is disclosed in WO 91/17244 as mentioned above (denoted EG I'). In Figure 3 is disclosed the amino acid sequence of a genetically truncated endoglucanase from *Humicola insolens* of 402 amino acid residues, this particular endoglucanase hereinafter being denoted EG I*. In Figure 4 is disclosed the amino acid sequence of a genetically truncated endoglucanase from *Fusarium oxysporum,* this particular endoglucanase hereinafter being denoted EGI-Fus. In figure 6 is disclose the amino acid sequence of an endoglucanase from *Myceliophthora thermophila.* Without being bound to the theory it is believed that an enhanced enzyme activity may be obtained by providing cellulases, especially endoglucanases, which essentially consists of a core. The cellulase may further comprise a C-terminal link, a "tail", which is relatively short, the short C-terminal link not contributing negatively to the enzyme activity. Thus it is believed that cellulases of the invention may be derived from known cellulases e.g. by "truncating" the C-terminal wholly or partly from the enzyme protein in question. The mentioned EG I* is thus derived from the known endoglucanase EG I (see WO 91/17244, Fig.14A-E) by eliminating the last 13 amino acids of the coding region. Furthermore, the amino acid residues in the positions 162, 203, 211 and 401, respectively, are substituted (162: Ser to Pro; 203: Val to Ala; 211: Val to Ala; 401: Val to Leu).

It is contemplated that cellulases which have an amino acid sequence being at least 60% homologous with the amino acid sequences listed in Figure 1, 3 and 4, respectively, also have enhanced activity resulting in improved soil removal from fabrics.

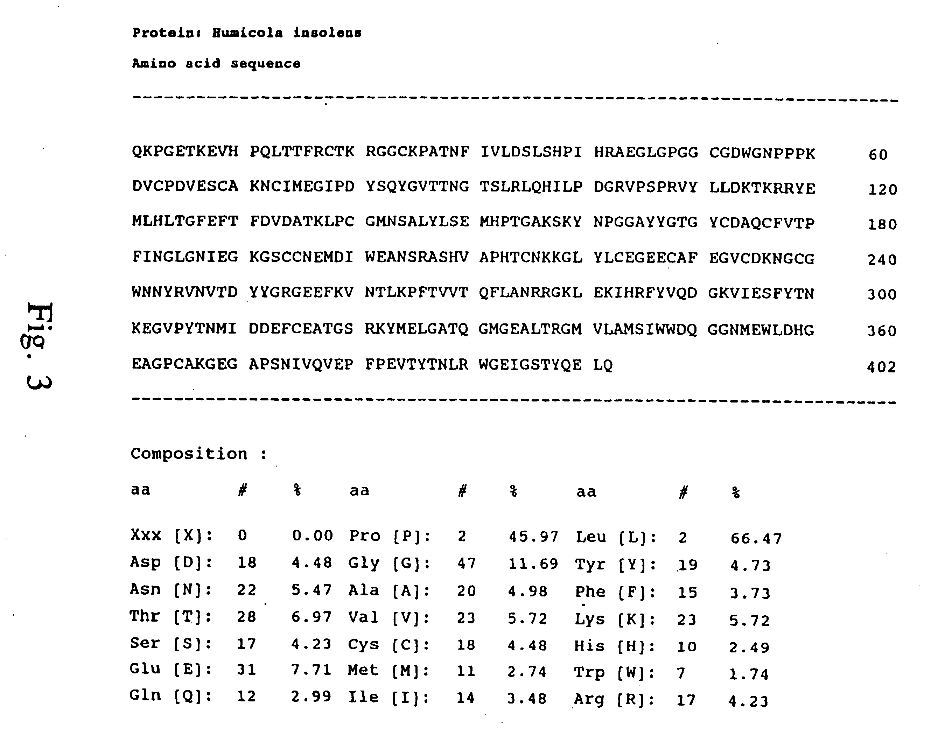

In Figure 2 is listed an alignment of the amino acid sequence of three known endoglucanases from *Humicola insolens* (denoted EGIHUM and having 415 amino acids), *Fusarium oxysporum* (denoted Cendofus and having 409 amino acids) and *Trichoderma reesei* (denoted Egltrite and having 435 amino acids), respectively.

Accordingly, the invention further relates to a cellulase which is derivable from a strain of *Myceliophthora thermophila* and comprises the amino acid residues 1-456, preferably the residues 1-420, more preferably the residues 21-420, listed in Figure 6 and optionally a C-terminal link consisting of 10 amino acids at the most, or a variant of said cellulase having an amino acid sequence being at least 60% homologous with said sequence, provided that the cellulase is different from the endoglucanase having the amino acid sequence listed in Figure 1. The pI of the cellulase from *Myceliophthora thermophila* was found to be 4.0.

Examples of variants of these cellulases are variants wherein one or more of the following amino acid residues are substituted: N89Q, N89Q+N247Q, H123N, T385N, Q399N, E202A, S37W+P39W.

Other useful variants are those wherein one or more of the following amino acid residues are substituted: M142E, K217A, K217A+K218T, R245G, I310D, E150Q, E334K, M198L.

Yet other useful cellulase variant are those wherein one or more of the following amino acid residues within 5Å of bound cellobiose are substituted:

R106x, Y145x, S345x, D34x, W51x, S104x, A143x, Y171x, D173x, Q175x, Y177x, W347x, where x is chosen to modify H-bonding potential and/or hydrophobic interaction with the substrate.

The activity of the present cellulases with respect to soil removal may be correlated to specific analytical methods.

Accordingly, the present invention further relates to a cellulase having high activity on cellotriose in the presence of a detergent matrix, high dispersing action on carbon black, and high alkaline activity on acid swollen cellulose at pH 8.

More specifically, the activity on cellotriose. (see below) in the presence of a detergent matrix corresponds to an apparent $k_{cal}$ at pH 8 of preferably at least 1 per sec; the dispersing action on carbon black at pH 10 corresponds to a delta value preferably of at least 0.20 measured at 582 nm for 5 mg/l of cellulase (see e.g. example 2); and the alkaline activity on acid swollen cellulose at pH 8 corresponds to an apparent $k_{cal}$ preferably of at least 10 per sec (see below).

EG I* (from *Humicola insolens;* see Fig. 3) has an apparent molecular weight (MW) of about 50kD due to glycosylation of the molecule. It is believed that the "true" MW is about 46kD. The pI of EG I* is at least about 0.4 lower than of EG I, since pI of EG I* is about 5.1-5.3 whereas pI of EG I is about 5.5-6.2.

EGI-Fus (from *Fusarium oxysporum;* see Fig. 4) has a apparent molecular weight (MW) of 48 kD; the amino acid composition gives 45 kD with 2 N glycosylation sites. The actual pI is above 9, and the theoretical pI is 9 which has been calculated based on the amino acid composition and using the pKa values from C. Tanford in Adv. Protein Chem. vol 17 pages 69-165, 1962. The molar extinction coefficient (based on the amino acid composition) has been calculated to 58180. It has been found that the stability of EGI-Fus is optimal at 50 degrees Celsius. The enzyme exhibits no activity above 60 degrees Celsius. The catalytic activity on cellotriose at pH 8,5 and 40°C has been calculated to 5,5 $K_{cal}$ per sec. Km is 0,5 mM. Further, the activity on CMC is about 315 ECU per mg protein. The activity of EGI-Fus can be inactivated by 3-epoxybutyl cellobioside; see e.g. G. Legler and E. Bause, Carbohydrate Research vol 28 (1973) page 45-52: Epoxyalkyl oligo (1-4) beta-D-Glucosides as active site directed inhibitors of cellulases.

The cellulases of the invention may be obtained from the microorganism in question by use of any suitable technique. For instance, a cellulase preparation may be obtained by fermentation of a microorganism and subsequent isolation of a cellulase containing preparation from the fermented broth or microorganism by methods known in the art, but more preferably by use of recombinant DNA techniques as known in the art. Such method normally comprises cultivation of a host cell transformed with a recombinant DNA vector capable of expressing and carrying a DNA sequence encoding the cellulase component in question, in a culture medium under conditions permitting the expression of the enzyme and recovering the enzyme from the culture.

Cloning a DNA sequence encoding a cellulase

The DNA sequence encoding a parent cellulase may be isolated from any cell or microorganism producing the cellulase in question by various methods, well known in the art. First a genomic-DNA and/or cDNA library should be constructed using chromosomal DNA or messenger RNA from the organism that produces the cellulase to be studied. Then, if the amino acid sequence of the cellulase is known, homologous, labelled oligonucleotide probes may be synthesized and used to identify cellulase-encoding clones from a genomic library of bacterial DNA, or from a fungal cDNA library. Alternatively, a labelled oligonucleotide probe containing sequences homologous to cellulase from another strain of bacteria or fungus could be used as a probe to identify cellulase-encoding clones, using hybridization and washing conditions of lower stringency.

Yet another method for identifying cellulase-producing clones would involve inserting fragments of genomic DNA into an expression vector, such as a plasmid, transforming cellulase-negative bacteria with the resulting genomic DNA library, and then plating the transformed bacteria onto agar containing a substrate for cellulase. Those bacteria containing cellulase-bearing plasmid will produce colonies surrounded by a halo of clear agar, due to digestion of the substrate by secreted cellulase.

Alternatively, the DNA sequence encoding the enzyme may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., The EMBO J. 3, 1984, pp. 801-805. According to the phosphoamidite method, oligonucleotides are synthesized, e.g. in an automatic DNA synthesizer, purified, annealed, ligated and cloned in appropriate vectors.

Finally, the DNA sequence may be of mixed genomic and synthetic, mixed synthetic and cDNA or mixed genomic and cDNA origin prepared by ligating fragments of synthetic, genomic or cDNA origin (as appropriate), the fragments corresponding to various parts of the entire DNA sequence, in accordance with standard techniques. The DNA sequence may also be prepared by polymerase chain reaction (PCR) using specific primers, for instance as described in US 4,683,202 or R.K. Saiki et al., Science 239, 1988, pp. 487-491.

Expression of cellulase variants

According to the invention, a mutated cellulase-coding sequence produced by methods described above, or any alternative methods known in the art, can be expressed, in enzyme form, using an expression vector which typically includes control sequences encoding a promoter, operator, ribosome binding site, translation initiation signal, and, optionally, a repressor gene or various activator genes. To permit the secretion of the expressed protein, nucleotides encoding a "signal sequence" may be inserted prior to the cellulase-coding sequence. For expression under the direction of control sequences, a target gene to be treated according to the invention is operably linked to the control sequences in the proper reading frame. Promoter sequences that can be incorporated into plasmid vectors, and which can support the transcription of the mutant cellulase gene, include but are not limited to the prokaryotic ß-lactamase promoter (Villa-Kamaroff, et al., 1978, Proc. Natl. Acad. Sci. U.S.A. 75:3727-3731) and the tac promoter (DeBoer, et al., 1983, Proc. Natl. Acad. Sci. U.S.A. 80:21-25). Further references can also be found in "Useful proteins from recombinant bacteria" in Scientific American, 1980, 242:74-94.

According to one embodiment B. subtilis is transformed by an expression vector carrying the mutated DNA. If expression is to take place in a secreting microorganism such as B. subtilis a signal sequence may follow the translation initiation signal and precede the DNA sequence of interest. The signal sequence acts to transport the expression product to the cell wall where it is cleaved from the product upon secretion. The term "control sequences" as defined above is intended to include a signal sequence, when is present.

In a currently preferred method of producing cellulase variants of the invention, a filamentous fungus is used as the host organism. The filamentous fungus host organism may conveniently be one which has previously been used as a host for producing recombinant proteins, e.g. a strain of Aspergillus sp., such as A. niger, A. nidulans or A. oryzae. The use of A. oryzae in the production of recombinant proteins is extensively described in, e.g. EP 238 023.

For expression of cellulase variants in Aspergillus, the DNA sequence coding for the cellulase variant is preceded by a promoter. The promoter may be any DNA sequence exhibiting a strong transcriptional activity in Aspergillus and may be derived from a gene encoding an extracellular or intracellular protein such as an amylase, a glucoamylase, a protease, a lipase, a cellulase or a glycolytic enzyme.

Examples of suitable promoters are those derived from the gene encoding A. oryzae TAKA amylase, Rhizomucor miehei aspartic proteinase, A. niger neutral $\alpha$-amylase, A. niger acid stable $\alpha$-amylase, A. niger glucoamylase, Rhizomucor miehei lipase, A. oryzae alkaline protease or A. oryzae triose phosphate isomerase.

In particular when the host organism is A. oryzae, a preferred promoter for use in the process of the present invention is the A. oryzae TAKA amylase promoter as it exhibits a strong transcriptional activity in A. oryzae. The sequence of the TAKA amylase promoter appears from EP 238 023.

Termination and polyadenylation sequences may suitably be derived from the same sources as the promoter.

The techniques used to transform a fungal host cell may suitably be as described in EP 238 023.

To ensure secretion of the cellulase variant from the host cell, the DNA sequence encoding the cellulase variant may be preceded by a signal sequence which may be a naturally occurring signal sequence or a functional part thereof or a synthetic sequence providing secretion of the protein from the cell. In particular, the signal sequence may be derived from a gene encoding an Aspergillus sp. amylase or glucoamylase, a gene encoding a Rhizomucor miehei lipase or protease, or a gene encoding a Humicola cellulase, xylanase or lipase. The signal sequence is preferably derived from the gene encoding A. oryzae TAKA amylase, A. niger neutral $\alpha$-amylase, A. niger acid-stable $\alpha$-amylase or A. niger glucoamylase.

The medium used to culture the transformed host cells may be any conventional medium suitable for growing Aspergillus cells. The transformants are usually stable and may be cultured in the absence of selection pressure. However, if the transformants are found to be unstable, a selection marker introduced into the cells may be used for selection.

The mature cellulase protein secreted from the host cells may conveniently be recovered from the culture medium by well-known procedures including separating the cells from the medium by centrifugation or filtration, and precipitating proteinaceous components of the medium by means of a salt such as ammonium sulphate, followed by chromatographic procedures such as ion exchange chromatography, affinity chromatography, or the like.

For example, EGI-Fus (from *Fusarium oxysporum,* Fig. 4) was produced by *Aspergillus oryzae* after transformation with a plasmid containing the disclosed sequence and using the normal taka promotor and AMG terminator. Fermentation of the transformed *A. oryzae* strain gave a yield of 380 ECU per ml. The fermentation broth was purified by filtration and concentration using ultrafiltration. The concentrate was adjusted to pH 5 and applied to a high performance S-Sepharose column equilibrated with 50 mM sodium acetate, pH 5.0. The enzyme bound and was eluted with a sodium choride salt gradient. The pure endoglucanase (EGI-Fus) was eluted with 0.5 M sodium chloride.

**Detergent Compositions**

According to the invention, the cellulase of the invention may typically be a component of a detergent composition. As such, it may be included in the detergent composition in the form of a non-dusting granulate, a stabilized liquid, or a protected enzyme. Non-dusting granulates may be produced, e.g., as disclosed in US 4,106,991 and 4,661,452 (both to Novo Industri A/S) and may optionally be coated by methods known in the art. Examples of waxy coating materials are poly(ethylene oxide) products (polyethyleneglycol, PEG) with mean molar weights of 1000 to 20000, ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in patent GB 1483591. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in EP 238,216. Accordingly, the present invention further relates to a detergent additive comprising a cellulase of the present invention.

The detergent composition of the invention may be in any convenient form, e.g. as powder, granules, paste or liquid. A liquid detergent may be aqueous, typically containing up to 70% water and 0-30% organic solvent, or nonaqueous.

**EP 0 749 473 B1**

The detergent composition comprises one or more surfactants, each of which may be anionic, nonionic, cationic, or zwitterionic. The detergent will usually contain 0-50% of anionic surfactant such as linear alkylbenzenesulfonate (LAS), alpha-olefinsulfonate (AOS), alkyl sulfate (fatty alcohol sulfate) (AS), alcohol ethoxysulfate (AEOS or AES), secondary alkanesulfonates (SAS), alpha-sulfo fatty acid methyl esters, alkyl- or alkenylsuccinic acid, or soap. It may also contain 0-40% of nonionic surfactant such as alcohol ethoxylate (AEO or AE), carboxylated alcohol ethoxylates, nonylphenol ethoxylate, alkylpolyglycoside, alkyldimethylamine - oxide, ethoxylated fatty acid monoethanolamide, fatty acid monoethanolamide, or polyhydroxy alkyl fatty acid amide (e.g. as described in WO 92/06154).

The detergent composition may additionally comprise one or more other enzymes, such as amylase, lipase, cutinase, protease, other cellulases, peroxidase, and oxidase, e.g., laccase).

The detergent may contain 1-65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (e.g. SKS-6 from Hoechst). The detergent may also be unbuilt, i.e. essentially free of detergent builder.

The detergent may comprise one or more polymers. Examples are carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may contain a bleaching system which may comprise a $H_2O_2$ source such as perborate or percarbonate which may be combined with a peracid-forming bleach activator such as tetraacetylethylenediamine (TAED) or nonanoyloxybenzenesulfonate (NOBS). Alternatively, the bleaching system may comprise peroxyacids of, e.g., the amide, imide, or sulfone type.

The enzymes of the detergent composition of the invention may be stabilized using conventional stabilizing agents, e.g. a polyol such as propylene glycol or glycerol, a sugar or sugar alcohol, lactic acid, boric acid, or a boric acid derivative such as, e.g., an aromatic borate ester, and the composition may be formulated as described in, e.g., WO 92/19709 and WO 92/19708.

The detergent may also contain other conventional detergent ingredients such as, e.g., fabric conditioners including clays, foam boosters, suds suppressors, anti-corrosion agents, soil-suspending agents, anti-soil-redeposition agents, dyes, bactericides, optical brighteners, or perfume.

The pH (measured in aqueous solution at use concentration) will usually be neutral or alkaline, e.g. in the range of 7-11.

Particular forms of detergent compositions within the scope of the invention include:

1) A detergent composition formulated as a granulate having a bulk density of at least 600 g/l comprising

| | |
|---|---|
| Linear alkylbenzenesulfonate (calculated as acid) | 7 - 12% |
| Alcohol ethoxysulfate (e.g. $C_{12-18}$ alcohol, 1-2 EO) or alkyl sulfate (e.g. $C_{16-18}$) | 1 - 4% |
| Alcohol ethoxylate (e.g. $C_{14-15}$ alcohol, 7 EO) | 5 - 9% |
| Sodium carbonate (as $Na_2CO_3$) | 14 - 20% |
| Soluble silicate (as $Na_2O,2SiO_2$) | 2 - 6% |
| Zeolite (as $NaAlSiO_4$) | 15 - 22% |
| Sodium sulfate (as $Na_2SO_4$) | 0 - 6% |
| Sodium citrate/citric acid (as $C_6H_5Na_3O_7/C_6H_8O_7$) | 0 - 15% |
| Sodium perborate (as $NaBO_3.H_2O$) | 11 - 18% |
| TAED | 2 - 6% |
| Carboxymethylcellulose | 0 - 2% |
| Polymers (e.g. maleic/acrylic acid copolymer, PVP, PEG) | 0 - 3% |
| Enzymes (calculated as pure enzyme protein) | 0.0001 - 0.1% |
| Minor ingredients (e.g. suds suppressors, perfume, optical brightener, photobleach) | 0 - 5% |

2) A detergent composition formulated as a granulate having a bulk density of at least 600 g/l comprising

| | |
|---|---|
| Linear alkylbenzenesulfonate (calculated as acid) | 6 - 11% |

(continued)

| | |
|---|---|
| Alcohol ethoxysulfate (e.g. $C_{12-18}$ alcohol, 1-2 EO or alkyl sulfate (e.g. $C_{16-18}$) | 1 - 3% |
| Alcohol ethoxylate (e.g. $C_{14-15}$ alcohol, 7 EO) | 5 - 9% |
| Sodium carbonate (as $Na_2CO_3$) | 15 - 21% |
| Soluble silicate (as $Na_2O$, $2SiO_2$) | 1 - 4% |
| Zeolite (as $NaAlSiO_4$) | 24 - 34% |
| Sodium sulfate (as $Na_2SO_4$) | 4 - 10% |
| Sodium citrate/citric acid (as $C_6H_5Na_3O_7/C_6H_8O_7$) | 0 - 15% |
| Carboxymethylcellulose | 0 - 2% |
| Polymers (e.g. maleic/acrylic acid copolymer, PVP, PEG) | 1 - 6% |
| Enzymes (calculated as pure enzyme protein) | 0.0001 - 0.1% |
| Minor ingredients (e.g. suds suppressors, perfume) | 0 - 5% |

3) A detergent composition formulated as a granulate having a bulk density of at least 600 g/l comprising

| | |
|---|---|
| Linear alkylbenzenesulfonate (calculated as acid) | 5 - 9% |
| Alcohol ethoxylate (e.g. $C_{12-15}$ alcohol, 7 EO) | 7 - 14% |
| Soap as fatty acid (e.g. $C_{16-22}$ fatty acid) | 1 - 3% |
| Sodium carbonate (as $Na_2CO_3$) | 10 - 17% |
| Soluble silicate (as $Na_2O$,$2SiO_2$) | 3 - 9% |
| Zeolite (as $NaAlSiO_4$) | 23 - 33% |
| Sodium sulfate (as $Na_2SO_4$) | 0 - 4% |
| Sodium perborate (as $NaBO_3.H_2O$) | 8 - 16% |
| TAED | 2 - 8% |
| Phosphonate (e.g. EDTMPA) | 0 - 1% |
| Carboxymethylcellulose | 0 - 2% |
| Polymers (e.g. maleic/acrylic acid copolymer, PVP, PEG) | 0 - 3% |
| Enzymes (calculated as pure enzyme protein) | 0.0001 - 0.1% |
| Minor ingredients (e.g. suds suppressors, perfume, optical brightener) | 0 - 5% |

4) A detergent composition formulated as a granulate having a bulk density of at least 600 g/l comprising

| | |
|---|---|
| Linear alkylbenzenesulfonate (calculated as acid) | 8 - 12% |
| Alcohol ethoxylate (e.g. $C_{12-15}$ alcohol, 7 EO) | 10 - 25% |
| Sodium carbonate (as $Na_2CO_3$) | 14 - 22% |
| Soluble silicate (as $Na_2O$,$2SiO_2$) | 1 - 5% |
| Zeolite (as $NaAlSiO_4$) | 25 - 35% |
| Sodium sulfate (as $Na_2SO_4$) | 0 - 10% |
| Carboxymethylcellulose | 0 - 2% |
| Polymers (e.g. maleic/acrylic acid copolymer, PVP, PEG) | 1 - 3% |
| Enzymes (calculated as pure enzyme protein) | 0.0001 - 0.1% |
| Minor ingredients (e.g. suds suppressors, perfume) | 0 - 5% |

5) An aqueous liquid detergent composition comprising

| | |
|---|---|
| Linear alkylbenzenesulfonate (calculated as acid) | 15 - 21% |
| Alcohol ethoxylate (e.g. $C_{12-15}$ alcohol, 7 EO or $C_{12-15}$ alcohol, 5 EO) | 12 - 18% |
| Soap as fatty acid (e.g. oleic acid) | 3 - 13% |
| Alkenylsuccinic acid ($C_{12-14}$) | 0 - 13% |
| Aminoethanol | 8 - 18% |
| Citric acid | 2 - 8% |
| Phosphonate | 0 - 3% |
| Polymers (e.g. PVP, PEG) | 0 - 3% |
| Borate (as $B_4O_7$) | 0 - 2% |
| Ethanol | 0 - 3% |
| Propylene glycol | 8 - 14% |
| Enzymes (calculated as pure enzyme protein) | 0.0001 - 0.1% |
| Minor ingredients (e.g. dispersants, suds suppressors, perfume, optical brightener) | 0 - 5% |

6) An aqueous structured liquid detergent composition comprising

| | |
|---|---|
| Linear alkylbenzenesulfonate (calculated as acid) | 15 - 21% |
| Alcohol ethoxylate (e.g. $C_{12-15}$ alcohol, 7 EO, or $C_{12-15}$ alcohol, 5 EO) | 3 - 9% |
| Soap as fatty acid (e.g. oleic acid) | 3 - 10% |
| Zeolite (as $NaAlSiO_4$) | 14 - 22% |
| Potassium citrate | 9 - 18% |
| Borate (as $B_4O_7$) | 0 - 2% |
| Carboxymethylcellulose | 0 - 2% |
| Polymers (e.g. PEG, PVP) | 0 - 3% |
| Anchoring polymers such as, e.g., lauryl methacrylate/acrylic acid copolymer; molar ratio 25:1; MW 3800 | 0 - 3% |
| Glycerol | 0 - 5% |
| Enzymes (calculated as pure enzyme protein) | 0.0001 - 0.1% |
| Minor ingredients (e.g. dispersants, suds suppressors, perfume, optical brighteners) | 0 - 5% |

7) A detergent composition formulated as a granulate having a bulk density of at least 600 g/l comprising

| | |
|---|---|
| Fatty alcohol sulfate | 5 - 10% |
| Ethoxylated fatty acid monoethanolamide | 3 - 9% |
| Soap as fatty acid | 0 - 3% |
| Sodium carbonate (as $Na_2CO_3$) | 5 - 10% |
| Soluble silicate (as $Na_2O, 2SiO_2$) | 1 - 4% |
| Zeolite (as $NaAlSiO_4$) | 20 - 40% |
| Sodium sulfate (as $Na_2SO_4$) | 2 - 8% |
| Sodium perborate (as $NaBO_3 . H_2O$) | 12 - 18% |
| TAED | 2 - 7% |

(continued)

| | |
|---|---|
| Polymers (e.g. maleic/acrylic acid copolymer, PEG) | 1 - 5% |
| Enzymes (calculated as pure enzyme protein) | 0.0001 - 0.1% |
| Minor ingredients (e.g. optical brightener, suds suppressors, perfume) | 0 - 5% |

8) A detergent composition formulated as a granulate comprising

| | |
|---|---|
| Linear alkylbenzenesulfonate (calculated as acid) | 8 - 14% |
| Ethoxylated fatty acid monoethanolamide | 5 - 11% |
| Soap as fatty acid | 0 - 3% |
| Sodium carbonate (as $Na_2CO_3$) | 4 - 10% |
| Soluble silicate (as $Na_2O,2SiO_2$) | 1 - 4% |
| Zeolite (as $NaAlSiO_4$) | 30 - 50% |
| Sodium sulfate (as $Na_2SO_4$) | 3 - 11% |
| Sodium citrate (as $C_6H_5Na_3O_7$) | 5 - 12% |
| Polymers (e.g. PVP, maleic/acrylic acid copolymer, PEG) | 1 - 5% |
| Enzymes (calculated as pure enzyme protein) | 0.0001 - 0.1% |
| Minor ingredients (e.g. suds suppressors, perfume) | 0 - 5% |

9) A detergent composition formulated as a granulate comprising

| | |
|---|---|
| Linear alkylbenzenesulfonate (calculated as acid) | 6 - 12% |
| Nonionic surfactant | 1 - 4% |
| Soap as fatty acid | 2 - 6% |
| Sodium carbonate (as $Na_2CO_3$) | 14 - 22% |
| Zeolite (as $NaAlSiO_4$) | 18 - 32% |
| Sodium sulfate (as $Na_2SO_4$) | 5 - 20% |
| Sodium citrate (as $C_6H_5Na_3O_7$) | 3 - 8% |
| Sodium perborate (as $NaBO_3.H_2O$) | 4 - 9% |
| Bleach activator (e.g. NOBS or TAED) | 1 - 5% |
| Carboxymethylcellulose | 0 - 2% |
| Polymers (e.g. polycarboxylate or PEG) | 1 - 5% |
| Enzymes (calculated as pure enzyme protein) | 0.0001 - 0.1% |
| Minor ingredients (e.g. optical brightener, perfume) | 0 - 5% |

10) An aqueous liquid detergent composition comprising

| | |
|---|---|
| Linear alkylbenzenesulfonate (calculated as acid) | 15 - 23% |
| Alcohol ethoxysulfate (e.g. $C_{12-15}$ alcohol, 2-3 EO) | 8 - 15% |
| Alcohol ethoxylate (e.g. $C_{12-15}$ alcohol, 7 EO, or $C_{12-15}$ alcohol, 5 EO) | 3 - 9% |
| Soap as fatty acid (e.g. lauric acid) | 0 - 3% |
| Aminoethanol | 1 - 5% |

(continued)

| Sodium citrate | 5 - 10% |
|---|---|
| Hydrotrope (e.g. sodium toluensulfonate) | 2 - 6% |
| Borate (as $B_4O_7$) | 0 - 2% |
| Carboxymethylcellulose | 0 - 1% |
| Ethanol | 1 - 3% |
| Propylene glycol | 2 - 5% |
| Enzymes (calculated as pure enzyme protein) | 0.0001 - 0.1% |
| Minor ingredients (e.g. polymers, dispersants, perfume, optical brighteners) | 0 - 5% |

11) An aqueous liquid detergent composition comprising

| Linear alkylbenzenesulfonate (calculated as acid) | 20 - 32% |
|---|---|
| Alcohol ethoxylate (e.g. $C_{12-15}$ alcohol, 7 EO, or $C_{12-15}$ alcohol, 5 EO) | 6 - 12% |
| Aminoethanol | 2 - 6% |
| Citric acid | 8 - 14% |
| Borate (as $B_4O_7$) | 1 - 3% |
| Polymer (e.g. maleic/acrylic acid copolymer, anchoring polymer such as, e.g., lauryl methacrylate/ acrylic acid copolymer) | 0 - 3% |
| Glycerol | 3 - 8% |
| Enzymes (calculated as pure enzyme protein) | 0.0001 - 0.1% |
| Minor ingredients (e.g. hydrotropes, dispersants, perfume, optical brighteners) | 0 - 5% |

12) A detergent composition formulated as a granulate having a bulk density of at least 600 g/l comprising

| Anionic surfactant (linear alkylbenzenesulfonate, alkyl sulfate, alpha-olefinsulfonate, alpha-sulfo fatty acid methyl esters, alkanesulfonates, soap) | 25 - 40% |
|---|---|
| Nonionic surfactant (e.g. alcohol ethoxylate) | 1 - 10% |
| Sodium carbonate (as $Na_2CO_3$) | 8 - 25% |
| Soluble silicates (as $Na_2O, 2SiO_2$) | 5 - 15% |
| Sodium sulfate (as $Na_2SO_4$) | 0 - 5% |
| Zeolite (as $NaAlSiO_4$) | 15 - 28% |
| Sodium perborate (as $NaBO_3.4H_2O$) | 0 - 20% |
| Bleach activator (TAED or NOBS) | 0 - 5% |
| Enzymes (calculated as pure enzyme protein) | 0.0001 - 0.1% |
| Minor ingredients (e.g. perfume, optical brighteners) | 0 - 3% |

13) Detergent formulations as described in 1) - 12) wherein all or part of the linear alkylbenzenesulfonate is replaced by ($C_{12}$-$C_{18}$) alkyl sulfate.

14) A detergent composition formulated as a granulate having a bulk density of at least 600 g/l comprising

| ($C_{12}$-$C_{18}$) alkyl sulfate | 9 - 15% |
|---|---|
| Alcohol ethoxylate | 3 - 6% |

(continued)

| Polyhydroxy alkyl fatty acid amide | 1 - 5% |
|---|---|
| Zeolite (as $NaAlSiO_4$) | 10 - 20% |
| Layered disilicate (e.g. SK56 from Hoechst) | 10 - 20% |
| Sodium carbonate (as $Na_2CO_3$) | 3 - 12% |
| Soluble silicate (as $Na_2O,2SiO_2$) | 0 - 6% |
| Sodium citrate | 4 - 8% |
| Sodium percarbonate | 13 - 22% |
| TAED | 3 - 8% |
| Polymers (e.g. polycarboxylates and PVP= | 0 - 5% |
| Enzymes (calculated as pure enzyme protein) | 0.0001 - 0.1% |
| Minor ingredients (e.g. optical brightener, photo bleach, perfume, suds suppressors) | 0 - 5% |

15) A detergent composition formulated as a granulate having a bulk density of at least 600 g/l comprising

| ($C_{12}$-$C_{18}$) alkyl sulfate | 4 - 8% |
|---|---|
| Alcohol ethoxylate | 11 - 15% |
| Soap | 1 - 4% |
| Zeolite MAP or zeolite A | 35 - 45% |
| Sodium carbonate (as $Na_2CO_3$) | 2 - 8% |
| soluble silicate (as $Na_2O,2SiO_2$) | 0 - 4% |
| Sodium percarbonate | 13 - 22% |
| TAED | 1 - 8% |
| Carboxymethyl cellulose | 0 - 3% |
| Polymers (e.g. polycarboxylates and PVP) | 0 - 3% |
| Enzymes (calculated as pure enzyme protein) | 0.0001 - 0.1% |
| Minor ingredients (e.g. optical brightener, phosphonate, perfume) | 0 - 3% |

16) Detergent formulations as described in 1) - 15) which contain a stabilized or encapsulated peracid, either as an additional component or as a substitute for already specified bleach systems.

17) Detergent compositions as described in 1), 3), 7), 9) and 12) wherein perborate is replaced by percarbonate.

18) Detergent compositions as described in 1), 3), 7), 9), 12), 14) and 15) which additionally contain a manganese catalyst. The manganese catalyst may, e.g., be one of the compounds described in "Efficient manganese catalysts for low-temperature bleaching", Nature 369, 1994, pp. 637-639.

19) Detergent composition formulated as a nonaqueous detergent liquid comprising a liquid nonionic surfactant such as, e.g., linear alkoxylated primary alcohol, a builder system (e.g. phosphate), enzyme and alkali. The detergent may also comprise anionic surfactant and/or a bleach system.

The endoglucanase of the invention may be incorporated in concentrations conventionally employed in detergents. It is at present contemplated that, in the detergent composition of the invention, the endoglucanase may be added in an amount corresponding to 0.00001-1 mg (calculated as pure enzyme protein) of endoglucanase per liter of wash liquor.

In yet another aspect, the present endoglucanases may be used in fabric softeners, e.g. as described in Surfactant and Consumer Products, Ed. by J. Falbe, 1987, pp 295-296; Tenside Surfactants Detergents, 30 (1993), 6, pp 394-399; JAOCS, Vol. 61 (1984), 2, pp 367-376; EP 517 762; EP 123 400; WO 92/19714; WO 93/19147; US 5,082,578; EP 494 769; EP 544 493; EP 543 562; US 5,235,082; EP 568 297; EP 570 237.

The present invention also relates to a washing process wherein soiled fabric is treated with a cellulase of the invention or an endoglucanase derived from a strain of *Humicola insolens* and having the amino acid sequence listed in Figure 3 and an apparent molecular weight of about 50 kD measured in SDS-PAGE.

It is contemplated that, dependent on the specificity of the modified cellulase, it may be employed for one or possibly more of the applications mentioned above, i.e. in the baking industry, in the wine and juice industry, for animal feed, and in textile and papermaking pulp processing. In a particular embodiment, the enzyme preparation of the invention may comprise a combination of one or more modified cellulases with enzymes selected from the group consisting of unmodified or modified amylases, lipases, proteases, oxidoreductases and hemicellulases.

Pulp and paper applications

In the papermaking pulp industry, the cellulase and/or enzyme preparation according to the invention may be applied advantageously e.g. as follows:

- For debarking: pretreatment with the cellulase and/or enzyme preparation according to the invention may degrade the cambium layer prior to debarking in mechanical drums resulting in advantageous energy savings.

- For defibration: treatment of a material containing cellulosic fibers with the cellulase and/or enzyme preparation of the invention prior to refining or beating may result in reduction of the energy consumption due to the hydrolysing effect of the cellulase on the interfibre surfaces. Use of the cellulase and/or enzyme preparation of the invention may result in improved energy savings as compared to the use of unmodified enzymes, since it is believed that the modified cellulase may possess a higher ability to penetrate fibre walls.

- For fibre modification, i.e. improvement of fibre properties where partial hydrolysis across the fibre wall is needed which requires deeper penetrating enzymes (e.g. in order to make coarse fibers more flexible). Deep treatment of fibers has so far not been possible for high yield pulps e.g. mechanical pulps or mixtures of recycled pulps. This has been ascribed to the nature of the fibre wall structure that prevents the passage of enzyme molecules due to physical restriction of the pore matrix of the fibre wall. It is contemplated that the modified (i.e. derivatised) cellulases of the invention are capable of penetrating into the fibre wall.

- For drainage improvement. The drainability of papermaking pulps may be improved by treatment of the pulp with hydrolysing enzymes, e.g. cellulases. Use of the modified cellulase and/or enzyme preparation according to the invention may be more effective, e.g. result in a higher degree of loosening bundles of strongly hydrated micro-fibrils in the fines fraction (consisting of fibre debris) that limits the rate of drainage by blocking hollow spaces between fibers and in the wire mesh of the paper machine. The Canadian standard freeness (CSF) increases and the Schopper-Riegler drainage index decreases when pulp in subjected to cellulase treatment, see e.g. US patent 4,923,565; TAPPI T227, SCAN C19:65 which are hereby incorporated by reference.

- For inter fibre bonding. Hydrolytic enzymes are applied in the manufacture of papermaking pulps for improving the inter fibre bonding. The enzymes rinse the fibre surfaces for impurities e.g. cellulosic debris, thus enhancing the area of exposed cellulose with attachment to the fibre wall, thus improving the fibre-to-fibre hydrogen binding capacity. This process is also referred to as dehornification. Paper and board produced with a cellulase containing enzyme preparation according to the invention may have an improved strength or a reduced grammage, a smoother surface and an improved printability. These improvements are believed to be a result of the improved penetrability of the modified/derivatised enzyme(s).

- For enzymatic deinking. Partial hydrolysis of recycled paper during or upon pulping by use of hydrolysing enzymes such as cellulases are known to facilitate the removal and agglomeration of ink particles. Use of a modified cellulase and/or enzyme preparation according to the invention may give a more effective loosening of ink from the surface structure due to a better penetration of the enzyme molecules into the fibrillar matrix of the fibre wall, thus softening the surface whereby ink particles are effectively loosened. The agglomeration of loosened ink particles are also improved, due to a more efficient hydrolysis of cellulosic fragments found attached to ink particles originating from the fibres.

The treatment of lignocellulosic pulp may, e.g., be performed as described in WO 91/14819, WO 91/14822, WO 92/17573 and WO 92/18688.

Textile applications

In another embodiment, the present invention relates to use of the modified cellulase and/or enzyme preparation according to the invention in the bio-polishing process. Bio-Polishing is a specific treatment of the yarn surface which improves fabric quality with respect to handle and appearance without loss of fabric wettability. The most important effects of Bio-Polishing can be characterized by less fuzz and pilling, increased gloss/luster, improved fabric handle, increased durable softness and altered water absorbency. Bio-Polishing usually takes place in the wet processing of the manufacture of knitted and woven fabrics. Wet processing comprises such steps as e.g. desizing, scouring, bleaching, washing, dying/printing and finishing. During each of these steps, the fabric is more or less subjected to mechanical action. In general, after the textiles have been knitted or woven, the fabric proceeds to a desizing stage, followed by a scouring stage, etc. Desizing is the act of removing size from textiles. Prior to weaving on mechanical looms, warp yarns are often coated with size starch or starch derivatives in order to increase their tensile strength. After weaving, the size coating must be removed before further processing the fabric in order to ensure a homogeneous and wash-proof result. It is known that in order to achieve the effects of Bio-Polishing, a combination of cellulolytic and mechanical action is required. It is also known that "super-softness" is achievable when the treatment with cellulase is combined with a conventional treatment with softening agents. It is contemplated that use of the modified cellulase and/or enzyme preparation of the invention for bio-polishing of cellulosic fabrics is advantageous, e.g. a more thorough polishing can be achieved. Bio-Polishing may be obtained by applying the method described e.g. in WO 93/20278.

Degradation of plant material

In yet another embodiment, the present invention relates to use of a modified cellulase and/or enzyme preparation according to the invention for degradation of plant material e.g. cell walls.
It is contemplated that the modified cellulase and/or enzyme preparation of the invention is useful in the preparation of wine, fruit or vegetable juice in order to increase yield. Cellulases according to the invention may also be applied for enzymatic hydrolysis of various plant cell-wall derived materials or waste materials, e.g. agricultural residues such as wheat-straw, corn cobs, whole corn plants, nut shells, grass, vegetable hulls, bean hulls, spent grains, sugar beet pulp, and the like. The plant material may be degraded in order to improve different kinds of processing, facilitate purification or extraction of other components like purification of beta-glucan or beta-glucan oligomers from cereals, improve the feed value, decrease the water binding capacity, improve the degradability in waste water plants, improve the conversion of e.g. grass and corn to ensilage, etc.
In a preferred embodiment of the invention, the cellulase is an endoglucanase. The cellulolytic activity of endoglucanase is determined relative to an analytical standard and may be expressed in the unit ECU.
Cellulolytic enzymes hydrolyse CMC, thereby decreasing the viscosity of the incubation mixture. The resulting reduction in viscosity may be determined by a vibration viscosimeter (e.g. MIVI 3000 from Sofraser, France).
Determination of the cellulolytic activity, measured in terms of ECU, may be determined according to the analysis method AF 301.1 which is available from the Applicant upon request.
The ECU assay quantifies the amount of catalytic activity present in the sample by measuring the ability of the sample to reduce the viscosity of a solution of carboxy-methylcellulose (CMC). The assay is carried out at 40°C, pH 7.5 using a relative enzyme standard for reducing the viscosity of the CMC substrate.

**Cellulase activity** on **cellotriose**

The cellulase activity on cellotriose, in terms of $k_{cal} \cdot S^{-1}$, was determined by a coupled assay:

$$\text{Cellotriose} \rightarrow \text{Glucose} + \text{Cellobiose (cat.: cellulase)}$$

$$\text{Glucose} + O_2 + H_2O \rightarrow \text{Gluconate} + H_2O_2 \text{ (cat.: Glucoseoxidase)}$$

$$H_2O_2 + \text{ABTS}^R \rightarrow \text{ABTS}^{Ox} \text{ (cat.: Peroxidase)}$$

which is followed spectrophotometrically at 418 nm (maximum absorbance of $\text{ABTS}^{Ox}$ at 418 nm).

*Method:*

The GOD-Perid Test Kit (available from Boehringer Mannheim, art. 124 036) was used. The buffer-enzyme solution in the test kit was dissolved in 500 ml milli Q water. pH of the solution was adjusted to 8.5 (NaOH).

80 mg of ABTS[R] (available from Boehringer Mannheim, art. 756 407) was dissolved in 10 ml GOD-Perid corresponding to a total concentration of ABTS[R] of 10 mg/ml.

A substrate stock solution of 5 mmole (2.52 mg/ml) of cellotriose (available from Merck art. 24741) in water was prepared. Diluted solutions in water corresponding to 1000 µmole, 500 µmole, 376 µmole, 250 µmole, 100 µmole and 60 µmole were prepared.

The reaction mixture was prepared by mixing 1 part of substrate solution with 1 part of GOD-Perid.

A solution of the cellulase enzyme to be determined in a concentration of 1.0 - 3.0 µmole was prepared.

50 µl of enzyme solution and 450 µl of reaction mixture were mixed.

The measurements were carried out on a HP 8452A Diode Array Spectrophotometer thermostated at 40°C, 1 cm cuvette, at a wavelength of 418 nm. The reaction was followed by measuring the oxidation af ABTS every 20 sec for 600 sec in total.

*Calculations:*

The cellulase activity on cellotriose, in terms of $k_{cal} \cdot s^{-1}$, was calculated from a Lineweaver-Burk plot (a plot of 1/V versus 1/[S]): the slope and the intersection were determined by linear regression analysis.

The following constants were used for the calculations:

Cellulase: $\varepsilon = 66,310$ $M^{-1} \bullet cm^{-1}$
ABTS$^{Ox}$ : $\varepsilon \doteq 0.0323$ $\mu mole^{-1} \bullet cm^{-1}$

For EG I' from *Fusarium oxysporum* (Figure 4), the catalytic activity on cellotriose at pH 8,5 and 40°C was calculated to 5,5 $K_{cal}$ pr. sec. $K_m$ was 0.5 mM.

## Determination of alkaline cellulase activity on amorphous cellulose

Method:

Substrate preparation: 20 gram acid-swollen AVICEL• stock solution (see below for a preparation which can be stored for one month) was centrifuged for 20 min. at 5000 rpm., the supernatant was poured off, and the sediment was resuspended in 30 ml of buffer. Then centrifuged for 20 min. at 5000 rpm, the supernatant was poured off, and the sediment was resuspended in buffer to a total of 30 g.. This corresponds to a substrate concentration of 10 g AVICEL/litre.

Buffer: 0.1M Barbital at pH 8.5 or 0.1M Glycine at pH 10.0

Enzyme solution:

The enzymes were diluted to an activity of 0.5 S-CEVU/ml at pH 8.5 or 0.75 S-CEVU/ml at pH 10.0.

Reagents:

2% NaOH, PHBAH-reagent: 1.5 g of p-hydroxy benzoic acid hydrazide and 5.0 g sodium tartrate was dissolved in 100 ml of 2% NaOH.

The substrate, the buffer and the enzyme solution were mixed as follows:

| Substrate µl | Buffer µl | Enzyme sol. µl | Subst. conc. (final) g/l |
|---|---|---|---|
| 50 | 1950 | 500 | 0.20 |
| 125 | 1875 | 500 | 0.50 |
| 250 | 1750 | 500 | 1.00 |
| 500 | 1500 | 500 | 2.00 |
| 750 | 1250 | 500 | 3.00 |

(continued)

| Substrate μl | Buffer μl | Enzyme sol. μl | Subst. conc. (final) g/l |
|---|---|---|---|
| 1000 | 1000 | 500 | 4.00 |

The substrate/buffer solution was preheated for 5 min at 40°C. Then the enzyme solution was added and the solution was whirlmixed for 5 sec., followed by incubation for 20 min. at 40°C.

The reaction was stopped by adding 500 μl 2% NaOH solution, followed by whirlmixing for 5 sec.

The samples were centrifuged for 20 min. at 5000 rpm.

1000 μl of supernatant was transferred from the test tubes to new test tubes, and 500 μl PHBAH-reagent was added, followed by boiling for 10 min.

The test tubes were cooled in ice water.

The absorbance of the samples were measured on a spectrophotometer at 410 nm.

Standard glucose curve:

A stock solution containing 300 mg/l was diluted to 5, 10, 15 and 25 mg/l.

1000 μl of the diluted standards were mixed with 500 μl of PHBAH-reagent, and were treated as the other samples, see above.

Determination of activity.

The release of reducing glucose equivalent was calculated using the standard curve.

The enzyme concentration was calculated using the molar absorbance of 66310 ($\varepsilon$) for the EG I endoglucanase. The $K_m$, $V_{max}$ and $K_{cal}$ was calculated from a Lineweaver-Burk plot using different substrate concentrations.

The molar absorbance of the cellulase variants having substituted tyrosines and tryptophanes was adjusted accordingly using a absorbance value for tryptophane of 5690 ($\varepsilon$) and for tyrosine of 1280 ($\varepsilon$) and cystein 120 ($\varepsilon$).

The extinction coefficients ($\varepsilon$) are disclosed in Gill,S.C. and Hippel, P.H.: Calculation of protein extinction coefficients from amino acid sequence data; Analytical Biochemistry vol 182, (319-326), (1989).

Each of the tested cellulases was purified to high homogeneity giving a single band in SDS-PAGE analysis (the ratio $A_{280}/A_{260}$ was checked as being above 1.5).

Preparation of Acid swollen cellulose:

Materials:

5 g Avicel•. (Art. 2331 Merck)
150 ml 85% Ortho-phosphoric-acid. (Art. 573 Merck)
400 ml Acetone. (Art. 14 Merck)
1.3 l Deionized water (Milli Q)
1 l glass beaker
1 l glass filter funnel
2 l suction flask
Ultra Turrax Homogenizer

Procedure:

The Acetone and the phosphoric-acid was cooled on ice.

The 5 g. Avicel• was moistened with water, then 150 ml of ice cold 85% ortho-phosphoric-acid was added, and the mixture was placed on ice bath with weak stirring for 1 h. 100 ml of ice cold acetone was added with stirring, followed by transfer of the mixture to a glass filter funnel, followed by washing with 3 x 100 ml ice cold acetone and dry suction after each washing.

The filter cake was washed with 2 x 500 ml water and sucked as dry as possible after each wash.

The filter cake was resuspended to a total volume of 300 ml and blended to homogeneity (using the Ultra Turrax Homogenizer).

The resulting product was stored in a refrigerator.

The following result was obtained

EG I* from *Humicola insolens* (Figure 3):

$k_{cal}$ at 8.5: 16 per sec
$k_{cal}$ at 10: 12 per sec

The extinction coefficient was 66310.

EGI-Fus from *Fusarium oxysporum* (Figure 4):

$k_{cal}$ at 8.5, 40°C: 16 per sec ($k_m$ 8g/l)
$k_{cal}$ at 10, 40°C: 4 per sec ($k_m$ 8g/l)

The molar extinction coefficient was 58180, calculated based on the amino acid composition.

## EXAMPLE 1

### Multi cycle terg-o-tometer test EG I* versus EG I

The example illustrates the superior soil removal effects of EG I* (truncated EG I, 402 amino acids, see Figure 3) versus EG I (415 amino acids, WO 91/17244 Fig. 14A-E). In the example EMPA 101 swatches have been used as soil removal tracers (carbon black/olive oil).

The following detergent composition was used:

|  | % by weight |
|---|---|
| LAS, (Nansa 1169/p) | 10.3 |
| AES, (Berol 452) | 3.5 |
| SOAP (C18) | 0.5 |
| SOAP (C14) | 0.5 |
| AEO (Dobanol 25-7) | 6.4 |
| Sodiumxylenesulfonat | 5.1 |
| Ethanol | 0.7 |
| MPG | 2.7 |
| Glycerol | 0.5 |
| Sodium sulphate | 0.40 |
| Sodium carbonate | 2.7 |
| Sodium citrate | 4.4 |
| Citric acid | 1.5 |
| Water | Rest |

Testing procedure

The test was based on a 2 cycle wash test in a terg-o-tometer using the detergent composition described above in a 0.3% solution with 1mM Ca++.

| Agitation | 150 m/min |
|---|---|
| Temperature | 40°C |
| pH | 8.2 |
| Swatches | EMPA 101 |
|  | (2 swatches à 5X6 cm pr 100 ml) |
| Washing time | 20 minutes |
| Rinse | 10 minutes in tapwater |
| Drying | Line drying at room temperature |
| Repetitions | 2. |

Result

The soil removal result is given as Delta remmision R (Enzyme-treated versus blind) measured at 420 nm with an Elrepho apparatus (DataColor).

| Enzyme | R (8.D.) |
|---|---|
| | **Delta R** |
| No enzyme | 41.99 (0.41) |
| | 0 |
| EG I, 4 ECU/l | 42.00 (0.28) |
| | 0.01 |
| EG I, 8 ECU/l | 41.24 (0.45) |
| | -0.75 |
| EG I, 12 ECU/l | 42.69 (0.32) |
| | 0.71 |
| EG I, 20 ECU/l | 43.05 (0.32) |
| | 1.06 |
| EG I*, 4 ECU/l | 41.61 (0.37) |
| | -0.38 |
| EG I*, 8 ECU/l | 43.41 (0.39) |
| | 1.42 |
| EG I*, 12 ECU/l | 44.29 (0.35) |
| | 2.30 |
| EG I*, 20 ECU/l | 44.34 (0.47) |
| | 2.35 |

## EXAMPLE 2

### Carbon dispersing effect of EG I*

The particulate soil removing effect of endoglucanases is expected partly to be ascribed to the cleavage of glycosidic bonds in the cellulose matrix, but to some extent the enzyme may also provide a more non-specific cleaning effect, for instance, by improving the dispersability of the particulate soil.

In this test it is shown that EG I* (truncated EG I, 402 amino acids, see Figure 3) differs from EG I (415 amino acids, WO 91/17244 Fig. 14A-E) with respect to its ability to disperse active carbon.

Different amounts of purified EG I and EG I* were added into 10ml of a 1g/l suspension of active carbon (Norit) in 10mM Phosphate buffer, pH 10.

The mixtures were incubated for 30 minutes at 55°C and 150 rpm.

After incubation the samples were allowed to cool to ambient temperature and non-dispersed carbon was allowed to settle (no centrifugation) for 15 minutes. The amount of carbon that was dispersed was evaluated by measuring the $OD_{582nm}$ of the supernatant (at 582nm was found a peak maximum most likely resulting from scattering). Due to the nature of the experiment (inhomogeneous solutions, time dependence etc), the absolute $OD_{582nm}$ levels may vary among the tests carried out, whereas the relative levels usually may be conserved.

The table below show the results of obtained in the test:

| Enzyme | conc. (mg/l) | ΔOD (582nm) |
|---|---|---|
| EG I | 5 | 0.09 |
| | 10 | 0.30 |
| EG I* | 5 | 0.40 |
| | 10 | 0.49 |
| ΔOD (582nm) $=OD_{582nm}$ (with enzyme) $-OD_{582nm}$ (without enzyme) $OD_{582nm}$ (without enzyme) =0.716 | | |

The results show that EG I* differs significantly from EG I in terms of its carbon dispersion ability - at 5mg/l level the ΔOD (582nm) obtained with EG I* is four times as high as that obtained with EG I. Also it should be noted that the effect of EG I* is actually very large - the apparant level of detergency is increased by about 70% in the presence of 10mg/l EG I*.

**EXAMPLE 2**

**Tensile strength loss induced by cellulase**

Cellulases used for soil removal in detergents often gives rise to an increased fabric wear. This can be observed through a reduced tensile strength of the fabric.

In the present example three cellulases are compared: Celluzyme• (a known commercial cellulase preparation), EG I* from *H. insolens* (Figure 3) and EG I-Fus from *Fusarium* (Figure 4).

Celluzyme• is a multicomplex cellulase product from *Humicola insolens* used in detergents for soil removal and color clarification.

**Experimental:**

| | |
|---|---|
| Buffer: | 0.05 M Tris-HCl, pH 7.0, 1 mM CaCl$_2$ |
| Textile/cup: | 4 pcs. à 5 x 25 cm; woven fabric (app. 18 g) |
| Dosage: | 10000 ECU/l of Celluzymes®, *Humicola insolens* EGI PPC 4192, *Fusarium* EGI-161294/MChr. |
| Volume: | 100 ml |
| Time: | 7 days dark storage, 25 °C. |
| Rinse: | 10 min in deionized water. |
| Evaluation: | Tensile strength is measured as wet pull on Instron. |

The following results were obtained:

| | % tensile strength loss |
|---|---|
| Blind (no cellulase) | 0% ± 8% **) |
| Celluzyme | 41% |
| *H. insolens* EG I* | 8% |
| *Fusarium* EGI-Fus | -4% |

**) 0% pr. definition; 8% is relative standard deviation.

From the results it can be concluded that EG I* from *H. insolens* and EGI-Fus from *Fusarium oxysporum* are effective for soil removal but their use do not result in significant tensile strength loss in textile fabric.

**Claims**

1. An endoglucanase which is derivable from a strain of *Myceliophthora thermophila* and comprises the amino acid residues 21-420, listed in Figure 6 or a variant of said endoglucanase having an amino acid sequence being at least 80% identical with said sequence.

2. The endoglucanase according to claim 1 comprising the residues 1-420 listed in Figure 6, or a variant of said endoglucanase having an amino acid sequence being at least 80% identical with said sequence.

3. The endoglucanase according to claim 1 comprising the residues 1-456 listed in Figure 6, or a variant of said endoglucanase having an amino acid sequence being at least 80% identical with said sequence.

4. The endoglucanase according to claim 1 comprising a C-terminal link consisting of 10 amino acids at the most, or a variant of said endoglucanase having an amino acid sequence being at least 80% identical with said sequence.

**5.** The endoglucanase according to claims 2 or 3, having one or more insertions of between 1 and 25 amino acid residues.

**6.** The endoglucanase according to claim 5, having one or more insertions of between 1 and 20 amino acid residues.

**7.** A detergent composition comprising an endoglucanase according to any of the preceding claims and a surfactant.

**8.** The detergent composition according to claim 8, which further comprises one or more other enzymes, in particular amylases, lipases, proteases, cellulase components, peroxidases, and/or oxidases.

**9.** A washing process wherein soiled fabric is treated with an endoglucanase according to any of the claims 1-6.

**10.** The use of an endoglucanase according to any of the claims 1-6 in detergent compositions or in fabric softener compositions in an amount which is effective for soil removal.

**11.** The use of an endoglucanase according to any of the claims 1-6 for colour clarification of textile fabrics.

**12.** The use of an endoglucanase according to any of the claims 1-6 for preventing backstaining in washing of fabric.

**13.** The use of an endoglucanase according to any of the claims 1-6 for soil removal from textiles.

**14.** The use of an endoglucanase according to any of the claims 1-6 for bio-polishing of textiles.

**15.** The use of an endoglucanase according to any of the claims 1-6 for deinking of used paper.

**16.** The use of an endoglucanase according to any of the claims 1-6 for drainage improvement of paper pulp.

**17.** The use of an endoglucanase according to any of the claims 1-6 for defibration of paper pulp.

**18.** The use of an endoglucanase according to any of the claims 1-6 for debarking of paper pulp.

**19.** The use of an endoglucanase according to any of the claims 1-6 for removal of soil from textile, said use resulting in insignificant tensile strength loss in the textile.

**Patentansprüche**

**1.** Endoglucanase, die von einem Stamm von *Myceliophthora thermophila* erhältlich ist und die in Figur 6 aufgelisteten Aminosäurereste 21-420 umfasst, oder eine Variante der Endoglucanase mit einer Aminosäuresequenz, die zu mindestens 80% mit der Sequenz identisch ist.

**2.** Endoglucanase nach Anspruch 1, umfassend die in Figur 6 aufgelisteten Reste 1-420, oder eine Variante der Endoglucanase mit einer Aminosäuresequenz, die zu mindestens 80% mit der Sequenz identisch ist.

**3.** Endoglucanase nach Anspruch 1, umfassend die in Figur 6 aufgelisteten Reste 1-456, oder eine Variante der Endoglucanase mit einer Aminosäuresequenz, die zu mindestens 80% mit der Sequenz identisch ist.

**4.** Endoglucanase nach Anspruch 1, umfassend eine C-terminale Verknüpfung, die höchstens aus 10 Aminosäuren besteht, oder eine Variante der Endoglucanase mit einer Aminosäuresequenz, die zu mindestens 80% mit der Sequenz identisch ist.

**5.** Endoglucanase nach den Ansprüchen 2 oder 3 mit einer oder mehreren Einfügungen aus zwischen 1 und 25 Aminosäureresten.

**6.** Endoglucanase nach Anspruch 5 mit einer oder mehreren Einfügungen aus zwischen 1 und 20 Aminosäureresten.

**7.** Detergenzzusammensetzung, umfassend eine Endoglucanase nach einem der vorangehenden Ansprüche und ein oberflächenaktives Mittel.

**8.** Detergenzzusammensetzung nach Anspruch 8, die des Weiteren ein oder mehrere andere Enzyme, insbesondere Amylasen, Ligascn, Proteasen, Cellulasebestandteile, Peroxidasen und/oder Oxidasen umfasst.

**9.** Waschvorgang, wobei verschmutztes Gewebe mit einer Endoglucanase nach einem der Ansprüche 1-6 behandelt wird.

**10.** Verwendung einer Endoglucanase nach einem der Ansprüche 1-6 in Detergenzzusammensetzungen oder in Gewebeweichmacherzusammensetzungen in einer Menge, die zur Schmutzentfernung wirksam ist.

**11.** Verwendung einer Endoglucanase nach einem der Ansprüche 1-6 zur Farbklärung von Textilgeweben.

**12.** Verwendung einer Endoglucanase nach einem der Ansprüche 1-6 zur Verhinderung der erneuten Fleckenbildung beim Waschen von Gewebe.

**13.** Verwendung einer Endoglucanase nach einem der Ansprüche 1-6 zur Schmutzentfernung von Textilien.

**14.** Verwendung einer Endoglucanase nach einem der Ansprüche 1-6 zum biologischen Verfeinern von Textilien.

**15.** Verwendung einer Endoglucanase nach einem der Ansprüche 1-6 zum Entfärben von Altpapier.

**16.** Verwendung einer Endoglucanase nach einem der Ansprüche 1-6 zur Entwässerungsverbesserung von Papierzellstoff.

**17.** Verwendung einer Endoglucanase nach einem der Ansprüche 1-6 zum Zerfasern von Papierzellstoff.

**18.** Verwendung einer Endoglucanase nach einem der Ansprüche 1-6 zum Entholzen von Papierzellstoff.

**19.** Verwendung einer Endoglucanase nach einem der Ansprüche 1-6 zur Entfernung von Schmutz von Textilien, wobei die Verwendung zu einem unerheblichen Reißfestigkeitsverlust in Textilien führt.

**Revendications**

**1.** Endoglucanase qui peut être dérivée d'une souche de *Myceliophthora thermophila* et comporte les résidus d'acide aminé 21 à 420, indiqués sur la figure 6, ou un variant de ladite endoglucanase ayant une séquence d'acides aminés qui est identique au moins à 80 % avec ladite séquence.

**2.** Endoglucanase selon la revendication 1, comportant les résidus 1 à 420 indiqués sur la figure 6, ou un variant de ladite endoglucanase ayant une séquence d'acides aminés qui est identique au moins à 80 % avec ladite séquence.

**3.** Endoglucanase selon la revendication 1, comportant les résidus 1 à 456 indiqués sur la figure 6, ou un variant de ladite endoglucanase ayant une séquence d'acides aminés qui est identique au moins à 80 % avec ladite séquence.

**4.** Endoglucanase selon la revendication 1, comportant une liaison C-terminale constituée de 10 acides aminés au plus, ou un variant de ladite endoglucanase ayant une séquence d'acides aminés qui est identique au moins à 80 % avec ladite séquence.

**5.** Endoglucanase selon la revendication 2 ou 3, ayant une ou plusieurs insertions comprises entre 1 et 25 résidus d'acide aminé.

**6.** Endoglucanase selon la revendication 5, ayant une ou plusieurs insertions comprises entre 1 et 20 résidus d'acide aminé.

**7.** Composition détergente comportant une endoglucanase selon l'une quelconque des revendications précédentes et un tensioactif.

**8.** Composition détergente selon la revendication 7, qui comporte de plus une ou plusieurs autres enzymes, en particulier des amylases, des lipases, des protéases, des composants de cellulase, des peroxydases et/ou des oxydases.

9. Processus de lavage dans lequel un tissu sale est traité à l'aide d'une endoglucanase selon l'une quelconque des revendications 1 à 6.

10. Utilisation d'une endoglucanase selon l'une quelconque des revendications 1 à 6 dans des compositions détergentes ou dans des compositions d'adoucissement de tissu selon une quantité qui est efficace pour l'élimination de saleté.

11. Utilisation d'une endoglucanase selon l'une quelconque des revendications 1 à 6 pour une clarification de couleur de tissus textiles.

12. Utilisation d'une endoglucanase selon l'une quelconque des revendications 1 à 6 pour prévenir une déteinte lors d'un lavage de tissu.

13. Utilisation d'une endoglucanase selon l'une quelconque des revendications 1 à 6 pour l'élimination de saleté à partir de textiles.

14. Utilisation d'une endoglucanase selon l'une quelconque des revendications 1 à 6 pour un bio-polissage de textiles.

15. Utilisation d'une endoglucanase selon l'une quelconque des revendications 1 à 6 pour le désencrage de papier usé.

16. Utilisation d'une endoglucanase selon l'une quelconque des revendications 1 à 6 pour l'amélioration de drainage de pâte à papier.

17. Utilisation d'une endoglucanase selon l'une quelconque des revendications 1 à 6 pour le défribage de pâte à papier.

18. Utilisation d'une endoglucanase selon l'une quelconque des revendications 1 à 6 pour l'écorçage de pâte à papier.

19. Utilisation d'une endoglucanase selon l'une quelconque des revendications 1 à 6 pour l'élimination de saleté à partir d'un textile, ladite utilisation ayant pour résultat une perte de résistance à la traction insignifiante du textile.

Protein: Humicola insolens

Amino acid sequence

---

| | | | | | | | |
|---|---|---|---|---|---|---|---|
| QKPGETKEVH | PQLTTFRCTK | RGGCKPATNF | IVLDSLSHPI | HRAEGLGPGG | CGDWGNPPPK | DVCPDVESCA | 70 |
| KNCIMEGIPD | YSQYGVTTNG | TSLRLQHILP | DGRVPSPRVY | LLDKTKRRYE | MLHLTGFEFT | FDVDATKLPC | 140 |
| GMNSALYLSE | MHPTGAKSKY | NSGGAYYGTG | YCDAQCFVTP | FINGLGNIEG | KGSCCNEMDI | WEVNSRASHV | 210 |
| VPHTCNKKGL | YLCEGEECAF | EGVCDKNGCG | WNNYRVNVTD | YYGRGEEFKV | NTLKPFTVVT | QFLANRRGKL | 280 |
| EKIHRFYVQD | GKVIESFYTN | KEGVPYTNMI | DDEFCEATGS | RKYMELGATQ | GMGEALTRGM | VLAMSIWWDQ | 350 |
| GGNMEWLDHG | EAGPCAKGEG | APSNIVQVEP | FPEVTYTNLR | WGEIGSTYQE | LQ | | 402 |

---

Composition :

| aa | | # | % | aa | | # | % | aa | | # | % |
|---|---|---|---|---|---|---|---|---|---|---|---|
| Xxx | [X]: | 0 | 0.00 | Pro | [P]: | 23 | 5.72 | Leu | [L]: | 26 | 6.47 |
| Asp | [D]: | 18 | 4.48 | Gly | [G]: | 47 | 11.69 | Tyr | [Y]: | 19 | 4.73 |
| Asn | [N]: | 22 | 5.47 | Ala | [A]: | 18 | 4.48 | Phe | [F]: | 15 | 3.73 |
| Thr | [T]: | 28 | 6.97 | Val | [V]: | 25 | 6.22 | Lys | [K]: | 23 | 5.72 |
| Ser | [S]: | 18 | 4.48 | Cys | [C]: | 18 | 4.48 | His | [H]: | 10 | 2.49 |
| Glu | [E]: | 31 | 7.71 | Met | [M]: | 11 | 2.74 | Trp | [W]: | 7 | 1.74 |
| Gln | [Q]: | 12 | 2.99 | Ile | [I]: | 14 | 3.48 | Arg | [R]: | 17 | 4.23 |

Fig. 1

EP 0 749 473 B1

| | | |
|---|---|---|
| Egltrire | qQPGTSTPEVHPKLTTYKCTKSGGCVAQQTSVVLDwnyrwm------HNYNSCTVNGGVN | 54 |
| Cendofus | -QTPDKAKEQHPKLETYRCTKASGCKKQTNYIVADagihgir-----RSAGCGDWGQKPN | 54 |
| EGIHUM | -QKPGETKEVHPQLTTFRCTKRGGCKPATNFIVLDslshpihraeglGPGGCGDWGNPPP | 59 |

| | | |
|---|---|---|
| Egltrire | TTLCPDEATCGKNCFIEGV---DYAASGVTTSGSSLTMNQYMPSSsggyssVSPRLYLLD | 111 |
| Cendofus | ATACPDEASCAKNCILSGMdsnAYKNAGITTSGNKLRLQQLINNQl-----VSPRVYLLE | 109 |
| EGIHUM | KDVCPDVESCAKNCIMEGIp--DYSQYGVTTNGTSLRLQHILPDGrv----PSPRVYLLD | 113 |

| | | |
|---|---|---|
| Egltrire | SD-GEYVMLKLNGQELSFDVDLSALPCGENGSLYLSQMDENGGAN--QYNTAGANYGSGY | 168 |
| Cendofus | ENKKKYEMLHLTGTEFSFDVEMEKLPCGMNGALYLSEMPQDGGKStsRNSKAGAYYGAGY | 169 |
| EGIHUM | KTKRRYEMLHLTGFEFTFDVDATKLPCGMNSALYLSEMHPTGAKS--KYNPGGAYYGTGY | 171 |

| | | |
|---|---|---|
| Egltrire | CDAQCPVQTWRNGTLNTSHQGFCCNEMDILEGNSRANALTPHSC--------------TA | 214 |
| Cendofus | CDAQCYVTPFINGVGNIKGQGVCCNELDIWEANSRATHIAPHPCSkpglygctgdecgSS | 229 |
| EGIHUM | CDAQCFVTPFINGLGNIEGKGSCCNEMDIWEVNSRASHVVPHTCNkkglylcegeecaFE | 231 |

| | | |
|---|---|---|
| Egltrire | TACDSAĠCGFNPYGSGYKSYYGPGDtvdtsktftiitqfntdngspsgnlvsitrkyqqn | 274 |
| Cendofus | GICDKAGCGWNHNRINVTDFYGRGKqykvdstrkftvtsqfvankqgdlielhrhyiqdn | 289 |
| EGIHUM | GVCDKNGCGWNNYRVNVTDYYGRGEefkvntlkpftvvtqflanrrgklekihrfyvqdg | 291 |

| | | |
|---|---|---|
| Egltrire | gvdipsaqpggdtisscpsasay-----------GGLATMGKALSSGMVLVFSIWNDNS | 322 |
| Cendofus | kviesavvnisgppkinfindkycaatganeymrlGGTKQMGDAMSRGMVLAMSVWWSEG | 349 |
| EGIHUM | kviesfytnkegvpytnmiddefceatgsrkymelGATQGMGEALTRGMVLAMSIWWDQG | 351 |

| | | |
|---|---|---|
| Egltrire | QYMNWLDSGNAGPCSSTEGNPSNILANNPNTHVVFSNIRWGDIGSTTNSTAPPPPpasst | 382 |
| Cendofus | DFMAWLDQGVAGPCDATEGDPKNIVKVQPNPEVTFSNIRIGEIGSTSSVKAPAYPgphrl | 409 |
| EGIHUM | GNMEWLDHGEAGPCAKGEGAPSNIVQVEPFPEVTYTNLRWGEIGSTYQEVQKPKPkpghg | 411 |

| | | |
|---|---|---|
| Egltrire | tfsttrrssttssspsctqthwgqcggigysgcktctsgttcqysndyysqcl | 435 |
| Cendofus | ----------------------------------------------------- | 409 |
| EGIHUM | prsd------------------------------------------------- | 415 |

Fig. 2

Protein: Humicola insolens

Amino acid sequence

------------------------------------------------------------------------

| QKPGETKEVH | PQLTTFRCTK | RGGCKPATNF | IVLDSLSHPI | HRAEGLGPGG | CGDWGNPPPK | 60 |
| DVCPDVESCA | KNCIMEGIPD | YSQYGVTTNG | TSLRLQHILP | DGRVPSPRVY | LLDKTKRRYE | 120 |
| MLHLTGFEFT | FDVDATKLPC | GMNSALYLSE | MHPTGAKSKY | NPGGAYYGTG | YCDAQCFVTP | 180 |
| FINGLGNIEG | KGSCCNEMDI | WEANSRASHV | APHTCNKKGL | YLCEGEECAF | EGVCDKNGCG | 240 |
| WNNYRVNVTD | YYGRGEEFKV | NTLKPFTVVT | QFLANRRGKL | EKIHRFYVQD | GKVIESFYTN | 300 |
| KEGVPYTNMI | DDEFCEATGS | RKYMELGATQ | GMGEALTRGM | VLAMSIWWDQ | GGNMEWLDHG | 360 |
| EAGPCAKGEG | APSNIVQVEP | FPEVTYTNLR | WGEIGSTYQE | LQ | | 402 |

------------------------------------------------------------------------

Composition :

| aa | # | % | aa | # | % | aa | # | % |
|---|---|---|---|---|---|---|---|---|
| Xxx [X]: | 0 | 0.00 | Pro [P]: | 2 | 45.97 | Leu [L]: | 2 | 66.47 |
| Asp [D]: | 18 | 4.48 | Gly [G]: | 47 | 11.69 | Tyr [Y]: | 19 | 4.73 |
| Asn [N]: | 22 | 5.47 | Ala [A]: | 20 | 4.98 | Phe [F]: | 15 | 3.73 |
| Thr [T]: | 28 | 6.97 | Val [V]: | 23 | 5.72 | Lys [K]: | 23 | 5.72 |
| Ser [S]: | 17 | 4.23 | Cys [C]: | 18 | 4.48 | His [H]: | 10 | 2.49 |
| Glu [E]: | 31 | 7.71 | Met [M]: | 11 | 2.74 | Trp [W]: | 7 | 1.74 |
| Gln [Q]: | 12 | 2.99 | Ile [I]: | 14 | 3.48 | Arg [R]: | 17 | 4.23 |

Fig. 3

Fusarium ENDO a EG I fam-C MATURE

One letter aminoacid codecode mature protein sequence


```
QTPDKAKEQH PKLETYRCTK ASGCKKQTNY IVADAGIHGI RQKNGAGCGD
WGQKPNATAC PDEASCAKNC ILSGMDSNAY KNAGITTSGN KLRLQQLINN
QLVSPRVYLL EENKKKYEML HLTGTEFSFD VEMEKLPCGM NGALYLSEMP
QDGGKSTSRN SKAGAYYGAG YCDAQCYVTP FINGVGNIKG QGVCCNELDI
WEANSRATHI APHPCSKPGL YGCTGDECGS SGICDKAGCG WNHNRINVTD
FYGRGKQYKV DSTRKFTVTS QFVANKQGDL IELHRHYIQD NKVIESAVVN
ISGPPKINFI NDKYCAATGA NEYMRLGGTK QMGDAMSRGM VLAMSVWWSE
GDFMAWLDQG VAGPCDATEG DPKNIVKVQP NPEVTFSNIR IGEIGSTSSV
KAPAYPGPHR L
```

Fusarium ENDO a EG I fam-C MATURE
Composition :

| aa | | | # | % | aa | | | # | % | aa | | | # | % |
|---|---|---|---|---|---|---|---|---|---|---|---|---|---|---|
| Xxx | [X] | : | 0 | 0.00 | Pro | [P] | : | 20 | 4.87 | Leu | [L] | : | 21 | 5.11 |
| Asp | [D] | : | 21 | 5.11 | Gly | [G] | : | 45 | 10.95 | Tyr | [Y] | : | 17 | 4.14 |
| Asn | [N] | : | 28 | 6.81 | Ala | [A] | : | 32 | 7.79 | Phe | [F] | : | 9 | 2.19 |
| Thr | [T] | : | 22 | 5.35 | Val | [V] | : | 21 | 5.11 | Lys | [K] | : | 31 | 7.54 |
| Ser | [S] | : | 26 | 6.33 | Cys | [C] | : | 18 | 4.38 | His | [H] | : | 9 | 2.19 |
| Glu | [E] | : | 20 | 4.87 | Met | [M] | : | 11 | 2.68 | Trp | [W] | : | 6 | 1.46 |
| Gln | [Q] | : | 18 | 4.38 | Ile | [I] | : | 22 | 5.35 | Arg | [R] | : | 14 | 3.41 |

Fig. 4

EP 0 749 473 B1

```
SEQUENCE         10        20        30        40        50        60   Res

EG1, Fusariu  -QTPDKAKEQHPKLETYRCTKASGCKKQTNYIVADAGIHGIRQKN---GAGCGDWGQKPN   56
EG1, Humicol  -QKPGETKEVHPQLTTFRCTKRGGCKPATNFIVLDSLSHPIHRAEGLGPGCGDWGNPPP   59
EG1, T. rees  QQPGTSTPEVHPKLTTYKCTKSGGCVAQQTSVVLDWNYRWMHNYNS----CTVNGGV-N   54
CBH1, T.rees  XSACTLQSETHPPLTWQKCSSGGTCTQQTGSVVIDANWRWTHATNS--STNCYD-GNTWS   57


SEQUENCE         70        80        90       100       110       120   Res

EG1, Fusariu  ATACPDEASCAKNCILSGMDSNAYKN-AGITTSGNKLRLQQLI--N--NQLVSPRVYLL  110
EG1, Humicol  KDVCPDVESCAKNCIMEGIP--DYSQ-YGVTTNGTSLRLQHILP--D--GRVPSPRVYLL  112
EG1, T. rees  TTLCPDEATCGKNCFIEGV---DYAA-SGVTTSGSSLTMNQYMPSSSGYSSVSPRLYLL  110
CBH1, T.rees  STLCPDNETCAKNCCLDGA--AYASTYGVTTSGNSLSIGFVT---QSAQKNVGARLYLM  111


SEQUENCE        130       140       150       160       170       180   Res

EG1, Fusariu  EENKKKYEMLHLTGTEFSFDVEMEKLPCGMNGALYLSEMPQDGGKSTSRNSKAGAYYGAG  170
EG1, Humicol  DKTKRRYEMLHLTGFEFTFDVDATKLPCGMNSALYLSEMHPTGAKS--KYNPGAYYGTG  170
EG1, T. rees  DSD-GEYVMLKLNGQELSFDVDLSALPCGENGSLYLSQMDENGGAN--QYNTAGANYGSG  167
CBH1, T.rees  ASD-TTYQEFTLLGNEFSFDVDVSQLPCGLNGALYFVSMDADGGVSKYPTNTAGAKYGTG  170


SEQUENCE        190       200       210       220       230       240   Res

EG1, Fusariu  YCDAQCYV-TPFINGVGNIK-------GQGVCCNELDIWEANSRATHIAPHPC  215
EG1, Humicol  YCDAQCFV-TPFINGLGNIE-------GKGSCCNEMDIWEANSRASHVAPHTC  215
EG1, T. rees  YCDAQCPV-QTWRNGTLNTS-------HQGFCCNEMDILEGNSRANALTPHSC  212
CBH1, T.rees  YCDSQCPRDLKFINGQANVEGWEPSSNNANTGIGGHGSCCSEMDIWEANSISEALTPHPC  230
```

Fig. 5 (Part I)

SEQUENCE 250 260 270 280 290 300 Res

EG1, Fusariu  SKPGLYGCTGDECG-------SSGICDKAGCGWNHNRINVTDFYGRGKQYKVDSTRKFTV  268
EG1, Humicol  NKKGLYLCEGEECA-------FEGVCDKNGCGWNNYRVNVTDYYGREEFKVNTLKPFTV  268
EG1, T. rees  TAT---ACDSAGCG-----------------FNPYGSGYKSYYGPGDTV--DTSKTFTI  249
CBH1, T.rees  TTVGQEICEGDGCGGTYSDNRYGGTCDPDGCDWNPYRLGNTSFYGPGSSFTLDTTKKLTV  290

SEQUENCE 310 320 330 340 350 360 Res

EG1, Fusariu  TSQFVANKQ---GDLIELHRHYIQDNKVIESAVVNISGPPKINFINDKYCAATGA----N  321
EG1, Humicol  VTQFLANRR---GKLEKIHRFYVQDGKVIESFYTNKEGVPYTNMIDDEFCEATGS----R  321
EG1, T. rees  ITQFNTDNGSPSGNLVSITRKYQQNGVDIPSA--------QPGGDTISSCPSASA-----  296
CBH1, T.rees  VTQFETS-------GAINRYYVQNGVTFQQPNAEL-GSYSGNELNDDYCTAEEAEFGGS  341

SEQUENCE 370 380 390 400 410 420 Res

EG1, Fusariu  EYMRLGGTKQMGDAMSRGMVLAMSVWWSEGDFMAWLDQG------------VAGPCDATE  369
EG1, Humicol  KYMELGATQGMGEALTRGMVLAMSIWWDQGGNMEWLDHG-----------EAGPCAKGE  369
EG1, T. rees  ----YGGLATMGKALSSGMVLVFSIWNDNSQYMNWLDSG-----------NAGPCSSTE  340
CBH1, T.rees  SFSDKGGLTQFKKATSGGMVLVMSLWDDYYANMLWLDSTYPTNETSSTPGAVRGSCSTSS  401

SEQUENCE 430 440 450 460 470 480 Res

EG1, Fusariu  GDPKNIVKVQPNPEVTFSNIRIGEIGSTSSVKAPAYPGPHRL                    411
EG1, Humicol  GAPSNIVQVEPFPEVTYTNLRWGEIGSTYQEVQKPKPKPGHGPRSD               415
EG1, T. rees  GNPSNILANNPNTHVVFSNIRWGDIGSTTNSTAPPPPPASSTTFST               386
CBH1, T.rees  GVPAQVESQSPNAKVTFSNIKFGPIGSTGNPSG                             434

Fig. 5 (Part II)

EP 0 749 473 B1

Amino acid sequence of endoglucanase from Myceliophthora thermophila :
1
```
MGRGAAFLGL ASLLVGAAKA QTPGEGEEVH PQITTYRCTK   40
ADGCEEKTNY IVLDALSHPV HQVDNPYNCG DWGQKPNETA   80
CPDLESCARN CIMDPVSDYG RHGVSTDGTS LRLKQLVGGN  120
VVSPRVYLLD ETKERYEMLK LTGNEFTFDV DATKLPCGMN  160
SALYLSEMDA TGARSELNPG GATFGTGYCD AQCYVTPFIN  200
GLGNIEGKGA CCNEMDIWEA NARAQHIAPH PCSKAGPYLC  240
EGAECEFDGV CDKNGCAWNP YRVNVTDYYG EGAEFRVDTT  280
RPFSVVTQFR AGGDAGGGKL ESIYRLFVQD GRVIESYVVD  320
KPGLPPTDRM TDEFCAATGA ARFTELGAME AMGDALTRGM  360
VLALSIWWSE GDNMNWLDSG EAGPCDPDEG NPSNIIRVQP  400
DPEVVFSNLR WGEIGSTYES AVDGPVGKGK GKGKGKAPAG  440
DGNGKEKSNG KRFRRF                           456
```

The protein sequence starts at position 21.

The last 36 amino acid residues form a tail which will be removed by proteases.

# Fig. 6